(19) **Europäisches Patentamt / European Patent Office / Office européen des brevets**

(11) **EP 3 492 920 A1**

(12) **EUROPEAN PATENT APPLICATION**
published in accordance with Art. 153(4) EPC

(43) Date of publication:
**05.06.2019 Bulletin 2019/23**

(51) Int Cl.:
**G01N 33/68** [(2006.01)]  **C12M 1/34** [(2006.01)]
**G01N 27/62** [(2006.01)]  **G01N 21/77** [(2006.01)]

(21) Application number: **17834106.1**

(22) Date of filing: **19.07.2017**

(86) International application number:
**PCT/JP2017/026014**

(87) International publication number:
**WO 2018/021098 (01.02.2018 Gazette 2018/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **28.07.2016 JP 2016148973**

(71) Applicant: **Asahi Kasei Pharma Corporation
Tokyo 100-0006 (JP)**

(72) Inventor: **KOGUMA, Ryosuke
Tokyo 101-8101 (JP)**

(74) Representative: **Forstmeyer, Dietmar et al
BOETERS & LIECK
Oberanger 32
80331 München (DE)**

(54) **METHOD FOR MEASURING GLYCATED ALBUMIN**

(57)  An object of the present invention is to provide a method of making measured values, when they are different due to a difference in measurement method, agree with each other. The other object is to provide a method of measuring a glycated-albumin (GA) value traceable to a certified GA value conveniently at a low cost in a short period of time.

A method of measuring a GA value traceable to the certified GA value includes the following steps:
$A_1$) determining Regression equation III by steps including the following b), c), and $e_0$) to h); b) determining a GA concentration$_{[L]}$ (A) of two or more GA certified reference materials having respectively different certified GA values; c) measuring a GA-derived absorbance (D) of the two or more GA certified reference materials; $e_0$) creating Linear equation II$_0$ having an intercept of zero; f) determining two or more GA concentrations$_{[P]}$ (E) proportionate to the GA-derived absorbance (D) according to Equation II$_0$; g) determining two or more GA values (F); and h) creating Regression equation III.

Fig. 1

EP 3 492 920 A1

## Description

### Technical Field

The present invention relates to a method of measuring a glycated albumin (GA) value, more specifically, a method of measuring a GA value traceable to a certified GA value.

### Background Art

As a glycated protein serving as a very important indicator in the diagnosis and management of diabetes, hemoglobin A1c (HbA1c) that reflects an average blood glucose level for the previous one to two months or so, GA that reflects an average blood glucose level for the previous two weeks, and the like are introduced for routine tests. There is a plurality of measurement methods for each of these glycoproteins and an object to be measured is different, depending on the measurement method. In order to obtain a reliable value, the measurement method of HbA1c is standardized by International Federation of Clinical Chemistry (IFCC) and an NGSP value prevalent widely in the world is used also in Japan at present.

With respect to the measurement of a GA value, on the other hand, no reference measurement procedure has been established, making it impossible to construct a traceability system of a measured value. To overcome this problem, a project for "Establishment of reference procedure for GA measurement" was started by Japan Society of Clinical Chemistry (JSCC). As a result, a JSCC recommended procedure for GA measurement based on newly-developed isotope dilution mass spectrometry (ID-MS) was published in 2008 (Non-Patent Document 1). Furthermore, with the standardization in this JSCC recommended procedure, a serum-based certified reference material was put on the market in 2013 (supplied by Reference Material Institute for Clinical Chemistry Standards, name of certified reference material: Certified Reference material for Measurements of Glycated Albumin in Human Serum: JCCRM 611).

Thus, in standardization of a GA value, the JSCC recommended procedure using ID-MS has been established as a reference method and a value measured by this method becomes a certified GA value. In a clinical laboratory, the GA value is measured by an enzymatic method used in combination with measurement of absorbance which is a simple and inexpensive quantitative method (Non-Patent Document 1). This enzymatic method uses a calibrator exclusively used therefor (for example, Patent Documents 1 and 2).

Citation List

Patent Document

> Patent Document 1: WO2002/061119
> Patent Document 2: JP 2004-77457A

Non-Patent Document

Non-Patent Document 1: Clinical Chemistry, Takei, et al., 2008, 37: 178-191.

### Summary

### Technical Problem

Since the standardization of a GA value has been established, a GA value measured by the JSCC recommended procedure using ID-MS will hereinafter be used as a certified GA value, but ID-MS is a measurement method taking much time and requiring an expensive instrument. It is therefore necessary to continue to use the enzymatic method combined with absorbance measurement for the measurement of a GA value in clinical site and make the GA measured value by this enzymatic method traceable to the certified GA value. For example, it is necessary to establish a traceability system having, as an upper level standard, a certified reference material whose GA value measured by ID-MS is determined as a certified value and associating this certified reference material with a value of a reference material for the enzymatic method.

It has already been confirmed during standardization of a GA value that a certified GA value (mmol/mol) determined by ID-MS correlates with a GA value (%) determined by liquid chromatography (HPLC) using the enzymatic method, that is, a (glycated albumin area)/(albumin area) (%) measured by HPLC (Non-Patent Document 1).

The present inventor has found that when a GA value (mmol/mol) of a certified reference material (JCCRM 611) according to the JSCC recommended procedure is measured by the enzymatic method combined with absorbance measurement based on a calibration curve prepared using a reference material for measurement by the enzymatic method, the measured GA value does not always agree with the certified GA value of the certified reference material. In addition, the inventor has found that the degree of divergence between the certified GA value and the GA value (mmol/mol) determined by the enzymatic method combined with absorbance measurement is different depending on the level of the certified GA value described for the certified reference material. As a result, it has been revealed that even by comparison between the certified GA value of the certified reference material (JCCRM 611) and the GA value of a sample material each measured by the enzymatic method combined with absorbance measurement based on a calibration curve prepared using a reference material for the enzymatic method, the GA value of the sample material traceable to the certified GA value cannot be determined.

With the above-described problem in view, an object of the present invention is to provide a method capable of making, when measured values differ due to a difference in the measurement methods, the measured values agree with each other. The other object of the present invention is to provide a method of measuring a GA value traceable to the certified GA value conveniently at a low cost in a short period of time.

Solution to Problem

With a view to overcoming the above-described problem, the present inventor has proceeded with extensive study. As a result, it has been found that the above-described disagreement is attributable to a difference between an object to be measured by the enzymatic method (glycated amino acid / glycated peptide available by proteolysis of GA) and an object to be measured by ID-MS (all the "glycated lysine residues (DOF-Lys)" liberated by the hydrolysis of GA). It is very difficult to use the same material for the measurement by ID-MS and for the measurement by the enzymatic method because protease cannot completely decompose it into DOF-Lys moieties.

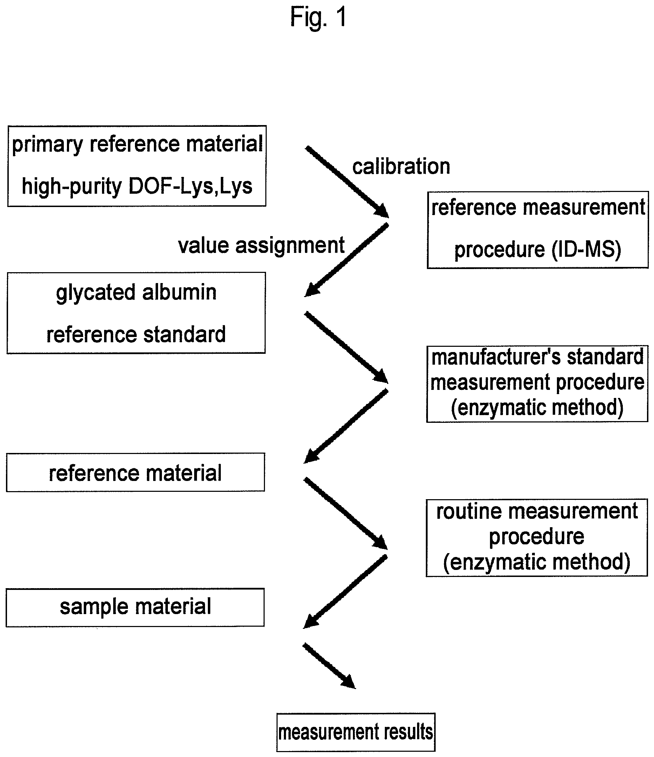

Under such a background, the present inventor has found a method of making a measured value of an object to be measured by the enzymatic method agree with a measured value of an object to be measured by ID-MS, establishing a traceability system (FIG. 1) having, as an upper level standard, a certified reference material whose GA value measured by ID-MS is determined as a certified value and associating this certified reference material with a value of a reference material for measurement by the enzymatic method. The present inventor has also found a method of measuring a GA value of a sample material traceable to a certified GA value conveniently at a low cost in a short period of time by the enzymatic method combined with absorbance measurement. The present invention is as follows.

[1] A method of measuring a glycated-albumin (GA) value traceable to a certified GA value, the method including the following steps:

Ao) a step of determining an equation for converting a measured value of a GA value of a sample material into a certified GA value of a GA certified reference material, a measured value of a GA concentration$_{[P]}$ of the sample material into a GA concentration$_{[L]}$ of the GA certified reference material, the measured value of the GA value of the sample material into a GA value set for the sample material from the certified GA value of the GA certified reference material, or the measured GA concentration$_{[P]}$ of the sample material into a GA concentration$_{[L]}$ set for the sample material from the GA concentration$_{[L]}$ of the GA certified reference material; and

F$_0$) a step of measuring a GA concentration$_{[P]}$ and an albumin concentration of the sample material by using a calibration curve prepared by using a reference material for which "a GA concentration$_{[P]}$ converted from the GA concentration$_{[L]}$ of the GA certified reference material" and "an albumin concentration converted from an albumin concentration of the GA certified reference material" are set; and determining a GA value traceable to the certified GA value according to the regression equation determined in Step A$_0$)

(in each step, the GA concentration$_{[L]}$ is a concentration of glycated lysine residues (DOF-Lys) and the GA concentration$_{[P]}$ is a concentration of a glycated amino acid or glycated peptide).

[2] A method of measuring a glycated-albumin (GA) value traceable to a certified GA value, the method including the following steps:
A$_1$) a step of determining Regression equation III by steps including the following b), c), and e$_0$) to h):

b) a step of determining respective GA concentrations$_{[L]}$ (A) of two or more GA certified reference materials having respectively different certified GA values according to the following Equation 1:

$$\text{GA concentration}_{[L]} \text{ (A)} = \text{(certified GA value (B) of GA certified}$$

$$\text{reference material)} \times \text{(albumin concentration (C) of GA certified reference}$$

$$\text{material)} \qquad \text{(Equation 1)}$$

c) a step of measuring respective GA-derived absorbances (D) of the two or more GA certified reference materials;

eo) a step of creating the following Linear equation $II_0$ having an intercept of zero:

$$D = \alpha_0 E \qquad \text{(Equation } II_0\text{)};$$

(wherein, $\alpha_0$ is an arbitrary positive value and E represents a GA concentration$_{[P]}$ (E) proportionate to the GA-derived absorbance (D)),

f) a step of determining two or more GA concentrations$_{[P]}$ (E) proportionate to the GA-derived absorbances (D) according to the Equation $II_0$;

g) a step of determining two or more GA values (F) according to the following Equation 2:

$$\text{GA value (F)} = (\text{GA concentration}_{[P]} \text{ (E)})/(\text{albumin concentration (C)})$$

$$\text{(Equation 2)};$$

and

h) a step of creating the following Regression equation III from the respective certified GA values (B) of the two or more GA certified reference materials and the two or more GA values (F) corresponding thereto:

$$B = \gamma F + \delta \qquad \text{(Regression equation III)}$$

(wherein in each of the steps, the GA concentration$_{[L]}$ is a concentration of glycated lysine residues (DOF-Lys) and the GA concentration$_{[P]}$ is a concentration of a glycated amino acid or a glycated peptide).

[2-1] The method described in [2], wherein the Equation $II_0$ is Equation II created by steps including the following Steps d) and e):

d) a step of creating the following Regression equation I from the two or more GA-derived absorbances (D) and the two or more GA concentrations$_{[L]}$ (A):

$$D = \alpha A + \beta \qquad \text{(Regression equation I)};$$

and

e) a step of creating the following Equation II by making the intercept of Regression equation I zero:

$$D = \alpha E \qquad \text{(Equation II)}.$$

[2-2] The method described in [2], wherein the Equation $II_0$ is Equation $II_1$ created by steps including the following Step $e_1$):

$e_1$) a step of creating the following Equation $II_1$ having an intercept of zero:

$$D = \alpha_1 E \qquad \text{(Equation } II_1\text{)}$$

(wherein $\alpha_1$ = [GA-derived absorbance (D) of one of the GA certified reference materials measured in Step c)]/[GA concentration$_{[L]}$ (A) of the corresponding GA certified reference material determined in Step b)]).

[3] The method described in any of [2] to [2-2] further comprising the following steps:

B) a step of measuring a GA-derived absorbance (G) of a reference material and determining a GA concentration$_{[P]}$ (H) corresponding thereto according to the Equation II$_0$, Equation II, or Equation II$_1$, and
C) a step of measuring an albumin-derived absorbance (I) of the reference material and determining an albumin concentration (J) of the reference material.

[4] The method described in [3], wherein Step C) includes the following Steps C1) to C3):

C1) a step of determining $\varepsilon$ that satisfies the following Equation 3 from an albumin-derived absorbance (K) of the GA certified reference material and the albumin concentration (C) of the GA certified reference material:

$$K=\varepsilon C \quad \text{(Equation 3)};$$

C2) a step of measuring the albumin-derived absorbance (I) of the reference material; and

C3) a step of determining the albumin concentration (J) of the reference material from the absorbance (I) according to the following Equation 3':

$$I=\varepsilon J \quad \text{(Equation 3')}.$$

[5] The method described in [3], wherein the method further includes, prior to Step b), the following Steps a1) to a3):

a1) a step of determining a molar certified albumin value (L) according to the following Equation 4:

$$\text{molar certified albumin value (L) = (certified albumin value of albumin}$$

$$\text{certified reference material)/(molecular weight of albumin)} \quad \text{(Equation 4)};$$

a2) a step of measuring an albumin-derived absorbance (M) of the albumin certified reference material; and

a3) a step of determining $\theta$ that satisfies the following Equation 5 from the molar certified albumin value (L) and the absorbance (M):

$$M=\theta L \quad \text{(Equation 5)};$$

and

Step (C) includes the following Steps C2) and C4):

C2) a step of measuring the albumin-derived absorbance (I) of the reference material; and

C4) a step of determining the albumin concentration (J) of the reference material from the absorbance (I) according to the following Equation 5':

$$I=\theta J \quad \text{(Equation 5')}.$$

[6] The method described in any of [2] to [5], further comprising the following Steps F1) to F4):

F1) a step of determining, from the GA-derived absorbance (D) of the GA certified reference material, a GA concentration[P] (N) corresponding thereto by using a calibration curve prepared by using the reference material;

F2) a step of determining, from the albumin-derived absorbance (K) of the GA certified reference material used in Step F1), an albumin concentration (O) corresponding thereto by using a calibration curve prepared by using the reference material;

F3) a step of determining a GA value (P) according to the following Equation 6:

$$\text{GA value (P) of GA certified reference material} = (\text{GA concentration}_{[P]}$$

$$(N))/(\text{albumin concentration (O)}) \quad \text{(Equation 6)};$$

and

F4) a step of determining a GA value (Q) traceable to the certified value of the GA certified reference material used in Step F1) while using the GA value (P) and the Regression equation III.

[7] A method of measuring a glycated-albumin (GA) value traceable to a certified GA value, the method including the following steps:

$A_2$) a step of determining Regression equation IV by carrying out steps including the following b), c), $e_0$), f), and i):

b) a step of determining respective GA concentrations[L] (A) of two or more GA certified reference materials having respectively different certified GA values according to the following Equation 1:

$$\text{GA concentration}_{[L]} \text{ (A)} = (\text{certified GA value (B) of GA certified}$$

$$\text{reference material}) \times (\text{albumin concentration (C) of GA certified reference}$$

$$\text{material}) \quad \text{(Equation 1)};$$

c) a step of measuring respective GA-derived absorbances (D) of two or more GA certified reference materials;

e0) a step of creating the following Linear Equation $II_0$ having an intercept of zero:

$$D = \alpha_0 E \quad \text{(Equation II}_0\text{)},$$

(wherein: $\alpha_0$ is an arbitrary positive value; and E is a GA concentration[P] (E) proportionate to the GA-derived absorbance (D));

f) a step of determining two or more GA concentrations[P] (E) proportionate to the GA-derived absorbances (D) according to the Equation $II_0$; and

i) a step of creating, from the two or more GA concentrations[L] (A) and GA concentrations[P] (E), the following Regression equation IV:

$$A = \kappa E + \lambda \quad \text{(Regression equation IV)}$$

(wherein in each of the above-described steps, the GA concentration[L] is a concentration of glycated lysine residues (DOF-Lys) and the GA concentration[P] is a concentration of a glycated amino acid or a glycated peptide).

[7-1] The method described in [7], wherein the Equation $II_0$ is Equation II created by steps including the following d) and e);

d) a step of creating, from the two or more GA-derived absorbances (D) and the two or more GA concentrations[L] (A), the following Regression equation I:

$$D = \alpha A + \beta \quad \text{(Regression equation I);}$$

and

e) a step of making the intercept of Regression equation I zero to create the following Equation II:

$$D = \alpha E \quad \text{(Equation II).}$$

[7-2] The method described in [7], wherein the Equation $II_0$ is Equation $II_1$ created by steps including the following $e_1$):

$e_1$) a step of creating the following equation $II_1$ having an intercept of zero:

$$D = \alpha_1 E \quad \text{(Equation II}_1\text{)}$$

(wherein $\alpha 1$ = [GA-derived absorbance (D) of one of the GA certified reference materials measured in Step c)]/[GA concentration$_{[L]}$ (A) of the corresponding GA certified reference material determined in Step b)]).

[8] The method described in any of [7] to [7-2], further including the following steps:

B) a step of measuring a GA-derived absorbance (G) of a reference material and determining a GA concentration$_{[P]}$ (H) corresponding thereto according to Equation $II_0$, Equation II, or Equation $II_1$; and

C) a step of measuring an albumin-derived absorbance (I) of the reference material and determining an albumin concentration (J) of the reference material.

[9] The method described in any of [7] to [8], further including the following steps:

F1) a step of determining, from the GA-derived absorbance (D) of the GA certified reference material, a GA concentration$_{[P]}$ (N) in the enzymatic method corresponding thereto by using a calibration curve prepared by using the reference material;

F2) a step of determining, from an albumin-derived absorbance (K) of the GA certified reference material used in Step F1), an albumin concentration (O) corresponding thereto by using a calibration curve prepared by using the reference material;

F5) a step of determining, from the GA concentration$_{[P]}$ (N), a GA concentration$_{[L]}$ (R) of the GA certified reference material according to the Regression equation IV; and

F6) a step of determining a GA value ($Q_1$') traceable to the certified value of the GA certified reference material used in Step F1) according to the following Equation 7:

$$\text{GA value } (Q_1\text{')} \text{ of GA certified reference material} = \frac{\text{GA concentration}_{[L]} (R)}{\text{albumin concentration } (O)} \quad \text{(Equation 7).}$$

[10] A method of measuring a glycated-albumin (GA) value traceable to a certified GA value, the method including the following steps:

$A_3$) a step of determining Regression equation V by steps including the following b) to $e_0$) and j) to o):

b) a step of determining respective GA concentrations$_{[L]}$ (A) of two or more GA certified reference materials having respectively different certified GA values according to the following Equation 1:

$$\text{GA concentration}_{[L]} (A) = (\text{certified GA value (B) of GA certified}$$

$$\text{reference material}) \times (\text{albumin concentration (C) of GA certified reference}$$

$$\text{material}) \quad \text{(Equation 1);}$$

c) a step of measuring respective GA-derived absorbances (D) of the two or more GA certified reference materials;

d) a step of creating, from the two or more GA-derived absorbances (D) and the two or more GA concentrations$_{[L]}$ (A), the following Regression equation I:

$$D = \alpha A + \beta \quad \text{(Regression equation I);}$$

eo) a step of creating the following Linear equation II$_0$ having an intercept of zero:

$$D = \alpha_0 E \quad \text{(Equation II}_0\text{)}$$

(wherein: $\alpha_0$ is an arbitrary positive value; and E represents a GA concentration$_{[P]}$ (E) proportionate to the GA-derived absorbance (D)),

j) a step of measuring respective GA-derived absorbances (S) of two or more sample materials and determining GA concentrations$_{[L]}$ (T) corresponding thereto according to the Regression equation I;

k) a step of measuring respective albumin-derived absorbances (U) of the two or more sample materials and determining albumin concentrations (V):

l) a step of determining, from the GA concentrations$_{[L]}$ (T), respective GA values$_1$ (W) of the two or more sample materials according to the following Equation 8:

$$\text{GA value}_1 (W) = (\text{GA concentration}_{[L]} (T))/(\text{albumin concentration (V)})$$

$$\text{(Equation 8);}$$

m) a step of determining, from the respective GA-derived absorbances (S) of the two or more sample materials, GA concentrations$_{[P]}$ (X) corresponding thereto according to the Equation II$_0$;

n) a step of determining, from the GA concentrations$_{[P]}$ (X), respective GA values$_2$ (Y) of the two or more sample materials according to the following Equation 9:

$$\text{GA value}_2 (Y) = (\text{GA concentration}_{[P]} (X))/(\text{albumin concentration (V)})$$

$$\text{(Equation 9);}$$

and

o) a step of creating, from the GA values$_1$ (W) of the two or more sample materials and the two or more GA values$_2$ (Y) corresponding thereto, the following Regression equation V:

$$W = \pi Y + \varphi \quad \text{(Regression equation V)}$$

(wherein in each step, the GA concentration$_{[L]}$ is a concentration of glycated lysine residues (DOF-Lys) and the GA concentration$_{[P]}$ is a concentration of a glycated amino acid or a glycated peptide).

[10-1] The method described in [10], wherein the Equation II$_0$ is Equation II created by steps including the following Step e):

e) a step of creating the following Equation II by making the intercept of Regression equation I zero:

$$D = \alpha E \quad \text{(Equation II)}.$$

[10-2] The method described in [10], wherein the Equation $II_0$ is Equation $II_1$ created by steps including the following Step $e_1$):

$e_1$) a step of creating the following Equation $II_1$ having an intercept of zero:

$$D = \alpha_1 E \quad \text{(Equation II}_1\text{)}$$

(wherein $\alpha_1$ = [GA-derived absorbance (D) of one of the GA certified reference materials measured in Step c)]/[GA concentration$_{[L]}$ (A) of the corresponding GA certified reference material determined in Step b)]).

[11] The method described in any of [10] to [10-2], further including the following steps:

B) a step of measuring a GA-derived absorbance (G) of a reference material and determining a GA concentration$_{[P]}$ (H) corresponding thereto according to the Equation $II_0$, Equation II, or Equation $II_1$; and

C) a step of measuring an albumin-derived absorbance (I) of the reference material and determining an albumin concentration (J) of the reference material.

[12] The method described in any of [10] to [11], further including the following steps:

F1') a step of determining, from the GA-derived absorbance (S) of the sample material, a GA concentration$_{[P]}$ (N') corresponding thereto by using a calibration curve prepared by using the reference material;

F2') a step of determining, from the albumin-derived absorbance (U) of the sample material used in Step F1'), an albumin concentration (O') corresponding thereto by using a calibration curve prepared by using the reference material;

F3') a step of determining a GA value (P') according to the following Equation 6':

$$\text{GA value (P') of sample material} = \text{(GA concentration}_{[P]} \text{ (N'))/(albumin concentration (O'))} \quad \text{(Equation 6');}$$

and

F4') a step of determining, by using the GA value (P') and Regression equation V, a GA value ($Q_2'$) traceable to a certified GA value$_1$ (W) with which the sample material used in Step F1') is assigned.

[13] A method of measuring a glycated-albumin (GA) value traceable to a certified GA value, the method including the following steps:

$A_4$) a step of determining Regression equation VI by steps including the following Steps b) to $e_0$), j), m), and p):

b) a step of determining respective GA concentrations$_{[L]}$ (A) of two or more GA certified reference materials having respectively different certified GA values according to the following Equation 1:

$$\text{GA concentration}_{[L]} \text{ (A)} = \text{(certified GA value (B) of GA certified}$$

$$\text{reference material)} \times \text{(albumin concentration (C) of GA certified reference}$$

$$\text{material)} \quad \text{(Equation 1)};$$

c) a step of measuring respective GA-derived absorbances (D) of the two or more GA certified reference materials;

d) a step of creating, from the two or more GA-derived absorbances (D) and the two or more GA concentrations$_{[L]}$ (A), Regression equation I:

$$D=\alpha A+\beta \quad \text{(Regression equation I)};$$

eo) a step of creating the following linear Equation $II_0$ having an intercept of zero:

$$D=\alpha_0 E \quad \text{(Equation } II_0\text{)}$$

(wherein, $\alpha_0$ is an arbitrary positive value and E is a GA concentration$_{[P]}$ (E) proportionate to the GA-derived absorbance (D));

j) a step of measuring respective GA-derived absorbances (S) of two or more sample materials and determining GA concentrations$_{[L]}$ (T) corresponding thereto according to the Regression equation I;

m) a step of determining, from the respective GA-derived absorbances (S) of the two or more sample materials, GA concentrations$_{[P]}$ (X) corresponding thereto according to the Equation $II_0$;

p) a step of creating, from the GA concentrations$_{[L]}$ (T) and GA concentrations$_{[P]}$ (X) of the two or more sample materials, Regression equation VI:

$$T=\psi X+\omega \quad \text{(Regression equation VI)}$$

(wherein in each step, the GA concentration$_{[L]}$ is a concentration of glycated lysine residues (DOF-Lys) and the GA concentration$_{[P]}$ is a concentration of a glycated amino acid or a glycated peptide).

[13-1] The method described in [13], wherein Equation $II_0$ is Equation II created by steps including the following Step e):
e) a step of creating Equation II by making an intercept of Regression equation I zero:

$$D=\alpha E \quad \text{(Equation II):}$$

[13-2] The method described in [13], wherein the Equation $II_0$ is Equation $II_1$ created by steps including the following Step $e_1$):
$e_1$) a step of creating the following Equation $II_1$ having an intercept of zero:

$$D=\alpha_1 E \quad \text{(Equation } II_1\text{)},$$

(wherein $\alpha_1$ = [GA-derived absorbance (D) of one of the GA certified reference materials measured in Step c)]/[GA concentration$_{[L]}$ (A) of the corresponding GA certified reference material determined in Step b)]).

[14] The method described in any of [13] to [13-2] further including the following steps:

B) a step of measuring a GA-derived absorbance (G) of a reference material and determining a GA concentration$_{[P]}$ (H) corresponding thereto according to the Equation II$_0$, Equation II, or Equation II$_1$; and
C) a step of measuring an albumin-derived absorbance (I) of the reference material and determining an albumin concentration (J) of the reference material.

[15] The method described in any of [13] to [14],
wherein the method further includes, prior to Step b), the following Steps a1) to a3):

a1) a step of determining a molar certified albumin value (L) according to the following Equation 4:

$$\text{molar certified albumin value (L)} = \text{(certified albumin value of albumin certified reference material)/(molecular weight of albumin)} \quad \text{(Equation 4)};$$

a2) a step of measuring an albumin-derived absorbance (M) of the albumin certified reference material; and
a3) a step of determining, from the molar certified albumin value (L) and the absorbance (M), $\theta$ that satisfies the following Equation 5:

$$M = \theta L \quad \text{(Equation 5)};$$

wherein Step A$_4$) further includes the following Step k):
k) a step of measuring respective albumin-derived absorbances (U) of the two or more sample materials to determine an albumin concentration (V);
Step A$_4$) further includes, after Steps j) and k), the following Step l):
l) a step of determining, from the GA concentrations$_{[L]}$ (T), respective GA values$_1$ (W) of the two or more sample materials according to the following Equation 8:

$$\text{GA value}_1\text{ (W)} = \text{(GA concentration}_{[L]}\text{ (T))/(albumin concentration (V))} \quad \text{(Equation 8)};$$

wherein the method further includes the following steps:

F1') a step of determining, from the GA-derived absorbance (S) of the sample material, a GA concentration$_{[P]}$ (N') in the enzymatic method corresponding thereto by using a calibration curve prepared by using the reference material;

F2') a step of determining, from the albumin-derived absorbance (U) of the sample material used in Step F1'), an albumin concentration (O') corresponding thereto by using a calibration curve prepared by using the reference material;

F5') a step of determining, from the GA concentration$_{[P]}$ (N'), a GA concentration$_{[L]}$ (R') of the sample material according to the Regression equation VI; and

F6') a step of determining a GA value (Q$_3$') traceable to a certified GA value$_1$ (W) set for the sample material used in Step F1') according to the following Equation 7':

$$\text{GA value (Q}_3\text{') of sample material} = \text{(GA concentration}_{[L]}\text{ (R'))/(albumin concentration (O'))} \quad \text{(Equation 7')}.$$

[16] The method described in any of [1] to [15], wherein three or more GA certified reference materials are used.

[17] The method described in any of [1] to [16], wherein the certified GA value is a certified GA value determined by isotope dilution mass spectrometry (ID-MS).

[18] The method described in any of [1] to [16], wherein the certified GA value is a certified GA value determined by isotope dilution mass spectrometry (ID-MS) and the GA certified reference material is JCCRM 611.

[19] A GA value measurement kit, including:

a regression equation selected from the group consisting of the Regression equation III determined by the method [2], [2-1] or [2-2], the Regression equation IV determined by the method [7], [7-1], or [7-2], the Regression equation V determined by the method [10], [10-1], or [10-2], and the Regression equation VI determined by the method [13],[13-1], or [13-2];

a GA measurement reagent;

an albumin measurement reagent; and

a reference material.

[20] An apparatus for measuring a GA value traceable to a certified glycated-albumin (GA) value, including:

an operation unit in which an equation selected from the group consisting of the Equation III determined by the method [2], [2-1], or [2-2], the Equation IV determined by the method [7], [7-1], or [7-2], the Equation V determined by the method [10], [10-1], or [10-2], and the Equation VI determined by the method [13],[13-1], or [13-2] is set;

a calibration curve preparation unit for preparing a calibration curve by using a reference material having a GA concentration$_{[P]}$ set according to the Equation II$_0$, Equation II, or Equation II$_1$, or a calibration curve preparation unit having incorporated therein the Equation II$_0$, Equation II, or Equation II$_1$; and

a measurement unit for measuring a GA value.

[21] A method of measuring a GA value traceable to a certified glycated-albumin (GA) value, including using 1) a reference material and 2) an operation program described below:

1) a reference material having a GA concentration$_{[P]}$ set according to the Equation II$_0$ created in Step e$_0$), the Equation II created in Step e), or the Equation II$_1$ created in Step e$_1$); and

2) an operation program having incorporated therein an equation selected from the group consisting of the Equation III determined by the method [2], [2-1], or [2-2], the Equation IV determined by the method [7], [7-1], or [7-2], the Equation V determined by the method [10], [10-1], or [10-2], and the Equation VI determined by the method [13], [13-1], or [13-2].

When an item number is cited as a range of, for example, from [2] to [5] and this range includes an item having a branch number such as [2-2], the item having a branch number such as [2-2] is also cited.

**Advantageous Effect of Invention**

According to the present invention, a method capable of making measured values agree with each other in the case where they do not agree with each other due to a difference in measurement method is provided.
For example, it becomes possible to measure a GA value of a sample material traceable to a certified GA value by using an enzymatic method capable of measuring it conveniently at a low cost in a short period of time. The present invention makes it possible to provide a method of measuring a GA value traceable to a certified GA value conveniently at a low cost in a short period of time.

**Brief Description of Drawings**

Fig. 1 shows a traceability system established in one aspect of the present embodiment.

Fig. 2 is Linear regression equation (Equation 5) created in Example 1 by plotting a molar certified albumin value (L) after unit conversion of an albumin certified reference material as the horizontal axis and an albumin-derived absorbance (M) as the vertical axis.

FIG. 3 is Linear regression equation (I) created in Example 1 by plotting a glycated albumin concentration$_{[L]}$ (A) of a glycated albumin certified reference material as the horizontal axis and a glycated albumin-derived absorbance (D) as the vertical axis.

FIG. 4 is Linear equation (II) set in Example 2 by moving Linear regression equation (I) in parallel to give an intercept of 0.

FIG. 5 is Linear regression equation (III) set in Example 2 by plotting a certified glycated-albumin value (B) of a glycated albumin certified reference material as the vertical axis and a glycated albumin value (F) as the horizontal axis.

FIG. 6 is Linear regression equation (Equation 3) set in Example 3 by plotting an albumin concentration (C) of a glycated albumin certified reference material as the horizontal axis and an albumin absorbance (K) as the vertical axis.

FIG. 7 is Linear equation (II$_{(d)}$) set in Example 6 obtained by changing the gradient of Linear regression equation (I) to give an intercept of 0 with reference to the glycated albumin concentration$_{[L]}$ (A) 330.0 $\mu$mol/L of the glycated albumin certified reference material HH obtained in Example 1.

FIG. 8 is Linear regression equation (III$_{(d)}$) set in Example 6 by plotting a certified glycated-albumin value (B) of a glycated albumin certified reference material as the vertical axis and a glycated albumin value (F$_{(d)}$) as the horizontal axis.

FIG. 9 is Linear regression equation (IV) set in Example 9 by plotting a glycated albumin concentration$_{[L]}$ (A) of a glycated albumin certified reference material as the vertical axis and the glycated albumin concentration$_{[P]}$ (E) as the horizontal axis.

FIG. 10 is Linear regression equation (V) set in Example 10 by plotting a glycated albumin value (W) (set value) of a sample material as the vertical axis and a glycated albumin value (Y) as the horizontal axis.

FIG. 11 is Linear regression equation (VI) set in Example 11 by plotting a glycated albumin concentration$_{[L]}$ (T) of a sample material as the vertical axis and a glycated albumin concentration$_{[P]}$ (X) as the horizontal axis.

FIG. 12 is Linear regression equation (I-2) set in Example 12 by plotting a glycated albumin concentration$_{[L]}$ (T-2) of a low-value (L) and high-value (H) reference materials as the horizontal axis and a glycated albumin-derived absorbance (S-2) as the vertical axis.

FIG. 13 is Linear equation (II-2) obtained in Example 12 by moving Linear regression equation (I-2) in parallel to give an intercept of 0.

FIG. 14 is Linear regression equation (III-2) set in Example 12 by plotting a glycated albumin value (W-2) (set value) of a low-value (L) and high-value (H) reference materials as the vertical axis and a glycated albumin value (Y-2) (measured value) as the horizontal axis.

FIG. 15 is Linear regression equation (I$_{(a)}$) set in Example 13 by plotting a glycated albumin concentration$_{[L]}$ (A$_{(a)}$) of a glycated albumin certified reference material as the horizontal axis and a glycated albumin-derived absorbance (D$_{(a)}$) as the vertical axis.

FIG. 16 is Linear equation (II$_{(a)}$) set in Example 13 by moving Linear regression equation (I$_{(a)}$) in parallel to give an intercept of 0.

FIG. 17 is Linear regression equation (III$_{(a)}$) set in Example 13 by plotting a certified glycated-albumin value (B) of a glycated albumin certified reference material as the vertical axis and a glycated albumin value (F$_{(a)}$) as the horizontal axis.

FIG. 18 is Linear regression equation (I$_{(b)}$) set in Example 14 by plotting a glycated albumin concentration$_{[L]}$ (A$_{(b)}$) of a glycated albumin certified reference material as the horizontal axis and a glycated albumin-derived absorbance (D$_{(b)}$) as the vertical axis.

FIG. 19 is Linear equation (II$_{(b)}$) set in Example 14 obtained by moving Linear regression equation (I$_{(b)}$) in parallel to give an intercept of 0.

FIG. 20 is Linear regression equation (III$_{(b)}$) set in Example 14 by plotting a certified glycated-albumin value (B) of a glycated albumin certified reference material as the vertical axis and a glycated albumin value (F$_{(b)}$) as the horizontal axis.

**Description of Embodiments**

The present invention will hereinafter be described in detail based on an embodiment of the present invention (also called "present embodiment"). The following embodiment is an example for describing the invention and is not intended to limit the invention only to this embodiment.

In one aspect, a method of measuring a GA value traceable to a certified glycated-albumin (GA) value according to the present embodiment includes a step (A$_1$) of determining Regression equation (III).

In the present embodiment, the term "glycated albumin" (GA which may also be called "glycoalbumin") means "albumin having glycated lysine residues (N$^\varepsilon$-(1-deoxy-D-fructose-1-yl)-L-lysine: DOF-Lys)" defined by the JSCC recommended procedure described specifically later.

In the present embodiment, the term "GA value" means a "molar ratio of (DOF-Lys of albumin): albumin" defined by the JSCC recommended procedure. The unit of the GA value is mmol/mol and can be calculated from the following equation:

$$\text{GA value} = (\text{GA concentration})/(\text{albumin concentration})$$

Here, the term "GA concentration" differs by a measurement principle. When measured using the enzymatic method, it means the concentration of a glycated amino acid or glycated peptide and in the present embodiment, it may be called "GA concentration$_{[P]}$". When measured using ID-MS, on the other hand, the GA concentration means a DOF-Lys concentration and in the present embodiment, it may be called "GA concentration$_{[L]}$".

In the present embodiment, the term "certified value" is a value indicated for a certified reference material and is measured by a reference measurement procedure. For example, a certified GA value is a value indicated for a GA certified reference material. In a preferred aspect of the present embodiment, the term "certified GA value" means a certified GA value by ID-MS and it is a GA value after value assignment and certification by the method (JSCC recommended procedure) described in Non-Patent Document 1.

In the present embodiment, the term "JSCC recommended procedure" for GA measurement means a method described in Non-Patent Document 1 and is a GA measurement method by which all DOF-Lys and lysine liberated after hydrolysis of a serum albumin fraction are measured simultaneously by ID-MS. The GA value measurement by the JSCC recommended procedure uses an expensive reagent and LC-MS, needs several days for one measurement and is a very complex method, because glucose does not bond to all of 59 lysine residues in GA to form DOF-Lys and DOF-Lys is also decomposed under hydrolysis conditions of a peptide bond of albumin so that stabilization of DOF-Lys or use of a stable isomer of DOF-Lys as an internal reference material becomes necessary.

In the present embodiment, the term "certified reference material" means a "reference material with a certificate, one or more of whose property values are certified by a procedure which establishes its traceability to an accurate realization of the unit in which the property values are expressed, and for which each certified value is accompanied by an uncertainty at a stated level of confidence" as defined by JIS Q 0030.

In the present embodiment, the term "GA certified reference material" means a certified reference material for measuring glycated albumin. Examples thereof include routine reference standard materials for glycoalbumin measurement provided by Reference Material Institute for Clinical Chemistry Standards (JCCRM 611 having three respectively different concentration levels M, H, and HH). The certified reference material may be a material for measuring glycated albumin agreed in the technical field based on the technical standards in the relevant area or at the time of use. In the preferred aspect of the present embodiment, three or more GA certified reference materials can be used as the GA certified reference materials. For example, three different concentration levels M, H, and HH of JCCRM 611 can be used.

In the present embodiment, the "albumin certified reference material" is a certified reference material for measuring albumin and it is not particularly limited insofar as it contains albumin and has a certified albumin value set therefor. Examples thereof include IFCC plasma protein international reference material (ERM-DA 470k/IFCC), Certified Reference Material for Measurement of Albumin in Human Serum JCCRM 613-1, and the like provided by Reference Material Institute for Clinical Chemistry Standards. The certified reference material may be a material for measuring albumin agreed in the technical field based on the technical standards in the relevant area or at the time of use.

The term "traceable" or "to become traceable" as used herein means, as defined in JIS Q 0030, "to become a measurement result or a property of a standard value which can be related to the stated reference which is usually a national standard or international standard through an unbroken chain of comparisons each having stated uncertainties." In one aspect of the present embodiment, "a predetermined measurement result becomes traceable" means that the measurement result links to a certified value of a certified reference material within a range of $\pm 5\%$.

In one aspect of the present embodiment, steps of determining Regression equation III include the following Steps b) to h).

Step b) is a step of determining a GA concentration$_{[L]}$ (A) of two or more GA certified reference materials having respectively different certified GA values according to Equation 1 (GA concentration$_{[L]}$ (A) = (certified GA value (B) of GA certified reference material) $\times$ (albumin concentration (C) of GA certified reference material). Here, the albumin concentration (C) of a GA certified reference material can be measured or determined by an arbitrary method and can be measured, for example, by an enzymatic method. Step b) can also be carried out using, as a reference value, the albumin concentration described in the certificate of the GA certified reference material.

Step c) is a step of measuring a GA-derived absorbance of two or more GA certified reference materials. The GA-derived absorbance is an absorbance based on GA obtained using a GA measurement reagent or the like and is: an absorbance determined by subtracting a blank absorbance from an absorbance obtained by actually measuring that of a sample material by using a measurement reagent or the like; or an absorbance of GA, which is separated by HPLC or the like, measured by using a GA measurement reagent or the like.

The absorbance can be measured using any known method. For example, it can be measured by referring to the method described in JP2001-54398A or the like and using a GA measurement reagent. As the GA measurement reagent, no particular limitation is imposed on it insofar as it contains an oxidation enzyme, a decomposition enzyme, a color developer, and the like and is capable of generating hydrogen peroxide by oxidation/decomposition of GA and determining a GA concentration from the absorbance of the color developer that has reacted with the hydrogen peroxide. The GA measurement reagent may contain a reference material which will be described later and may contain another component such as diluent or stabilizer. Each of the components may be stored in different containers. Specific examples of the reagent can refer to those described later in Examples and include, but not limited to, a GA reagent Lucica (trade mark) GA-L.

Step d) is a step of creating Regression equation (I):

$$D = \alpha A + \beta \quad \text{(Regression equation I)}$$

from the two or more GA-derived absorbances (D) and the two or more GA concentrations$_{[L]}$ (A). Step d) can be carried out, for example, by plotting the two or more GA-derived absorbances (D) as the vertical axis and the two or more GA concentrations$_{[L]}$ (A) as the horizontal axis to set a linear regression equation as shown later in Example.

Step $e_0$) is a step of creating Linear equation II$_0$ having an intercept of zero:

$$D = \alpha_0 E \quad \text{(Equation II}_0\text{)}.$$

Here, "E" represents a GA concentration$_{[P]}$ (E) proportionate to a GA-derived absorbance D. Step $e_0$) can be carried out by setting an arbitrary positive gradient $\alpha_0$. Step $e_0$) can be carried out, after Step c), without performing Step d).

Step e) is a step of creating Equation II:

$$D = \alpha E \quad \text{(Equation II)}$$

by making zero the intercept of Regression equation I obtained in Step d) as Equation $II_0$ having a gradient $\alpha_0$ in Step $e_0$). Here, "E" represents a GA concentration$_{[P]}$ (E) proportionate to a GA-derived absorbance D.

Step $e_1$) is a step of creating Equation $II_1$:

$$D=\alpha_1 E \quad \text{(Equation } II_1\text{)}$$

that passes through the origin and a point set from the GA-derived absorbance (D) of one of two or more GA certified reference materials measured in Step c) and the GA concentration$_{[L]}$ (A) of the corresponding GA certified reference material determined in Step b) and has an intercept of zero, as Equation $II_0$ having a gradient $\alpha_0$ in Step $e_0$). Here, the gradient $\alpha_1$ is [GA-derived absorbance (D) of one of the GA certified reference materials measured in Step c)]/[GA concentration$_{[L]}$ (A) of the corresponding GA certified reference material determined in Step b)] and E is a GA concentration$_{[P]}$ (E) proportionate to the GA-derived absorbance (D).

Step $e_1$) can be performed, for example, by plotting a GA-derived absorbance (D) of the GA certified reference material HH as the vertical axis and a GA concentration$_{[L]}$ (A) corresponding thereto as the horizontal axis and setting a linear equation passing through two points, that is, the point formed by them and the origin, as shown later in Example.

Step f) is a step of determining two or more GA concentrations$_{[P]}$ (E) proportionate to the GA-derived absorbances (D) according to Equation $II_0$. From the GA-derived absorbances (D) measured in Step c), GA concentrations$_{[P]}$ (E) corresponding thereto can be determined according to Equation $II_0$, that is, $D=\alpha_0 E$.

Step g) is a step of determining two or more GA values (F) according to Equation 2 (GA value (F) = GA concentration$_{[P]}$ (E)/albumin concentration (C)). Unit adjustment may be performed to obtain the GA value in a unit of mmol/mol. For example, when a GA concentration$_{[P]}$ ($\mu$mol/L) is divided by an albumin concentration ($\mu$mol/L), unit can be adjusted by multiplying by 1000.

Step h) is a step of creating Regression equation III:

$$B=\gamma F+\delta \quad \text{(Regression equation III)}$$

from the certified GA value (B) of the two or more GA certified reference materials and the two or more GA values (F) corresponding thereto. Step h) can be carried out, for example, by plotting the certified GA values (B) of the two or more GA certified reference materials as the vertical axis and the two or more GA values (F) corresponding thereto as the horizontal axis to set a linear regression equation, as shown later in Example.

Setting of Regression equation III as described above makes it possible to correlate the certified GA values of the GA certified reference materials and the GA values obtained by measurement of the GA certified reference materials by the enzymatic method.

The method of measuring a GA value traceable to a certified GA value in the present embodiment may further includes the following Steps B) and C):

B) a step of measuring a GA-derived absorbance (G) of a reference material and determining a GA concentration$_{[P]}$ (H) corresponding thereto according to Equation $II_0$, Equation II, or Equation $II_1$; and

C) a step of measuring an albumin-derived absorbance (I) of the reference material to determine an albumin concentration (J) of the reference material. By these steps, the reference material can be assigned with a concentration converted from the certified value. The term "albumin-derived absorbance" means an absorbance derived from albumin available by an albumin measurement reagent or the like. It is: an absorbance obtained by subtracting a blank absorbance from an absorbance of a sample material determined by the measurement using an albumin measurement reagent or the like; or an absorbance determined by the measurement of albumin which is isolated by HPLC or the like, by using an albumin measurement reagent or the like.

The term "reference material" as used herein means, as defined in JIS Q 0035:2008, a "material, sufficiently homogeneous and stable with respect to one or more specified properties, which has been established to be fit for its intended use in a measurement process". The reference material is also called a calibration sample, a calibrator, or a calibration curve preparation material and is a material used for preparation of a calibration curve. For example, as the reference material to be used in Step B) and Step C), a reference material for the measurement of a GA concentration and an albumin concentration by the enzymatic method can be used. Examples of such a reference material include GA-L calibrator (Asahi Kasei Pharma), a calibrator (Beijing Strong Biotechnologies, Inc.) Glycated albumin Calibrator (IVDLab CO., LTD), Glycated Serum Protein Assay Calibrators (Diazyme Laboratories), and glycated albumin calibrator (NINGBO MEDICALSYSTEM BIOTECHNOLOGY CO., LTD.).

In Step B), GA-derived absorbance (G) can be measured referring to Step c). From the absorbance (G) thus measured, a GA concentration$_{[P]}$ (H) corresponding thereto can be determined according to Equation II$_0$, Equation II, or Equation II$_1$, that is, G=$\alpha_0$H, G=$\alpha$H, or G=$\alpha_1$H.

In Step C), measurement of the albumin-derived absorbance (I) can be achieved using an arbitrary known method. For example, it can be performed using an albumin measurement reagent while referring to the method described inJP2009-159989A. The albumin measurement reagent is not limited insofar as it contains a dye and the like and permits measurement of an albumin concentration from the absorbance of a conjugate formed by conjugation of the dye and albumin. The albumin measurement reagent may contain the above-described reference material or may contain another component such as diluent or stabilizer. Examples of an albumin measurement method include a BCG method, a BCP method, and a modified BCP method. The BCG method is a measurement method making use of a measurement principle that albumin and bromocresol green (BCG) bind to each other to form a dye conjugate. The BCP method is a measurement method making use of a measurement principle that albumin and bromocresol purple (BCP) bind to each other to form a dye conjugate. It is characterized by low specificity to globulin, compared with the BCG method. The modified BCP method uses the BCP method as its basic principle and it contains, as a reagent, sodium dodecyl sulfate (SDS) and 5,5'-dithiobis(2-nitrobenzoic acid) (DTNB) in order to avoid an influence of human mercaptoalbumin having an SH group which is a cause of an error. As the dye, any dye such as bromocresol purple or bromocresol green is usable insofar as it binds to albumin. Specific examples of the reagent can be referred to Example described later. Usable examples include, but not limited to, Lucica (trade mark) GA-L which is an albumin measurement reagent for the modified BCP method and a glycated albumin measurement reagent (Beijing Strong Biotechnologies, Inc.) and Glycated serum albumin (IVDLab CO., LTD) and the like for the BCG method.

In one aspect, Step C) includes the following Steps C1) to C3):

C1) a step of determining, from an albumin-derived absorbance (K) of the GA certified reference material and the albumin concentration (C) of the GA certified reference material, $\epsilon$ that satisfies the following Equation 3:

$$K=\epsilon C \quad \text{(Equation 3)},$$

C2) a step of measuring an albumin-derived absorbance (I) of the reference material, and
C3) a step of determining, from the absorbance (I), an albumin concentration (J) of the reference material according to Equation 3':

$$I=\epsilon J \quad \text{(Equation 3')}$$

and by these steps, the albumin concentration (J) of the reference material can be determined.

The albumin-derived absorbance (K) of the GA certified reference material in Step C1) and the albumin-derived absorbance (I) of the reference material in Step C2) can be measured referring to Step C).

In another aspect, the method of measuring a GA value traceable to a certified GA value in the present embodiment includes, prior to Step b), the following Steps a1) to a3):

a1) a step of determining a molar certified albumin value (L) according to the following Equation 4:

$$\text{Molar certified albumin value (L)} = \text{(certified albumin value of albumin}$$

$$\text{certified reference material)/(molecular weight of albumin)} \quad \text{(Equation 4)};$$

a2) a step of measuring an albumin-derived absorbance (M) of the albumin certified reference material; and
a3) a step of determining, from the molar certified albumin value (L) and the absorbance (M), $\theta$ that satisfies the following equation 5:

$$M=\theta L \quad \text{(Equation 5)};$$

and

Step C) of this method includes the following Steps C2) and C4):

C2) a step of measuring an albumin-derived absorbance (I) of the reference material; and
C4) a step of determining, from the absorbance (I), an albumin concentration (J) of the reference material according to Equation 5':

$$I = \theta J \quad \text{(Equation 5'),}$$

by which the albumin concentration (J) of the reference material can be determined.

Step a1) can be performed referring to Example described later. For example, a molar certified albumin value ($\mu$mol/L) can be determined by dividing a certified albumin value (g/dL) of an albumin certified reference material by the molecular weight of albumin. Steps a2) and C2) can be performed referring to the above-described Step C). Albumin has a molecular weight of about 66000 and more specifically, a molecular weight described in known document can be used. For example, a molecular weight of 66438 described in Geisow MJ et. Al. In: Villafranca JJ, ed. Techniques in proteinchemistry. San Diego, CA: Academic Press, 1991: 576-572 may be used.

The method of measuring a GA value traceable to a certified GA value according to the present embodiment may further include the following Steps F1) to F4):

F1) a step of determining, from the GA-derived absorbance (D) of the GA certified reference material, a GA concentration$_{[P]}$ (N) corresponding thereto by using a calibration curve prepared by using a reference material;
F2) a step of determining, from the albumin-derived absorbance (K) of the GA certified reference material used in Step F1, an albumin concentration (O) corresponding thereto by using a calibration curve prepared by using the reference material;
F3) a step of determining a GA value (P) according to the following Equation 6:

$$\text{GA value (P) of GA certified reference material} = (\text{GA concentration}_{[P]}$$

$$(N))/(\text{Albumin concentration (O)}) \quad \text{(Equation 6);}$$

and
F4) a step of determining a GA value (Q) traceable to the certified value of the GA certified reference material used in Step F1) by using the GA value (P) and Regression equation III. By these steps, it is possible to confirm that a certified value can be calculated by measuring and converting the value of the certified reference material by using the calibration curve prepared by using the reference material.

The respective reference materials described in Step F1) and Step F3) are not particularly limited but when all of Steps B) and C) and Step F1) and Step F2) are performed in the method of the present embodiment, the reference material used in Steps B) and C) is the same as that used in Step F1) and Step F2).

The calibration curve can be prepared in Step F1) by measuring, in a manner similar to that described in Step c), a GA-derived absorbance of the reference material whose GA concentration$_{[P]}$ is specified.

The calibration curve can be prepared in Step F2) by measuring, in a manner similar to that described in Step C), an albumin-derived absorbance of the reference material whose albumin concentration is specified.

In Step F3), the GA value (P) of the GA certified reference material is determined by dividing the GA concentration$_{[P]}$ (N) determined in Step F1) by the albumin concentration (O) determined in Step F2). Here, unit adjustment may be performed to obtain the GA value in a unit of mmol/mol. For example, when a GA concentration$_{[P]}$ ($\mu$mol/L) is divided by an albumin concentration ($\mu$mol/L), unit can be adjusted by multiplying by 1000.

Further, in Step F4), a GA value (Q) traceable to the certified value of the GA certified reference material used in Step F1) can be determined by using the GA value (P) and Regression equation III, in other words, by applying it to $Q = \gamma F + \delta$. Establishment of a traceability system of a GA value using Regression equation III can be confirmed by confirming that the GA value (Q) obtained in Step F4) agrees with the certified value of the GA certified reference material used in Step F1). In short, it is confirmed that a GA value traceable to a certified GA value can be measured conveniently by using Regression equation III and the enzymatic method.

The above-described steps can be carried out in any order insofar as a measured value, regression equation, and the like necessary for each of the steps can be obtained. For example, Step b) and Step c) can be carried out in any order, Step B) and Step C) can be carried out in any order, and Step F1) and Step F2) can be carried out in any order.

The method of measuring a GA value traceable to a certified GA value according to the present embodiment can include, prior to Step b), the following Step a): a step of determining an albumin concentration (C) by measuring an albumin-derived absorbance (Z) of two or more GA certified reference materials having respectively different certified GA values.

Step a) can be carried out referring to the above-described Step C), for example, carried out using the BCG method, BCP method, or modified BCP method.

In one aspect, Step a) can include the following Steps a1) to a5):

a1) a step of determining a molar certified albumin value (L) according to the following Equation 4:

$$\text{Molar certified albumin value (L)} = \text{(certified albumin value of albumin}$$

$$\text{certified reference material)/(molecular weight of albumin)} \quad \text{(Equation 4);}$$

a2) a step of measuring an albumin-derived absorbance (M) of the albumin certified reference material;

a3) a step of determining, from the molar certified albumin value (L) and the absorbance (M), $\theta$ that satisfies the following Equation 5:

$$M=\theta L \quad \text{(Equation 5);}$$

a4) a step of measuring respective albumin-derived absorbances (Z) of two or more GA certified reference materials having respectively different certified GA values; and

a5) a step of determining, from the absorbances (Z), an albumin concentration (C) according to the following equation 5":

$$Z=\theta C \quad \text{(Equation 5").}$$

Steps a1) to a3) can be carried out as described above. Step a4) can be carried out referring to Step C).

In one aspect, the present embodiment also relates to a method of measuring a GA value traceable to a certified glycated-albumin (GA) value including Step $A_2$). Step $A_2$) is a step of determining Regression equation IV and it includes the following Steps b), c), $e_o$), f), and i):

b) a step of determining respective GA concentrations$_{[L]}$ (A) of two or more GA certified reference materials having respectively different certified GA values according to the following equation 1:

$$\text{GA concentration}_{[L]} \text{ (A)} = \text{(certified GA value (B) of GA certified}$$

$$\text{reference material)} \times \text{(albumin concentration (C) of GA certified reference}$$

$$\text{material)} \quad \text{(Equation 1);}$$

c) a step of measuring respective GA-derived absorbances (D) of two or more GA certified reference materials;
eo) a step of creating the following Linear Equation $II_0$ having an intercept of zero:

$$D=\alpha_0 E \quad \text{(Equation II}_0\text{)}$$

(wherein: $\alpha_0$ is an arbitrary positive value; and E is a GA concentration$_{[P]}$ (E) proportionate to the GA-derived absorbance (D));

f) a step of determining two or more GA concentrations$_{[P]}$ (E) proportionate to the GA-derived absorbances (D) according to Equation II$_0$; and

i) a step of creating, from the two or more GA concentrations$_{[L]}$ (A) and GA concentrations$_{[P]}$ (E), the following Regression equation IV:

$$A = \kappa E + \lambda \quad \text{(Regression equation IV)}.$$

In one aspect, the present embodiment also relates to a method of measuring a GA value traceable to a certified glycated-albumin (GA) value, wherein Equation II$_0$ in Step e$_0$) and Step f) included in Step A$_2$) is Equation II created by steps including the following Step d) and Step e):

d) a step of creating, from the two or more GA-derived absorbances (D) and the two or more GA concentrations$_{[L]}$ (A), Regression equation I:

$$D = \alpha A + \beta \quad \text{(Regression equation I)};$$

and

e) a step of making the intercept of Regression equation I zero to create Equation II:

$$D = \alpha E \quad \text{(Equation II)}.$$

In one aspect, the present embodiment also relates to a method of measuring a GA value traceable to a certified glycated-albumin (GA) value in which Equation II$_0$ in Step e$_0$) and Step (f) included in Step A$_2$) is Equation II$_1$ created by a step including the following Step e$_1$):

e$_1$): a step of creating the following Equation II$_1$ having an intercept of zero:

$$D = \alpha_1 E \quad \text{(Equation II}_1\text{)}$$

(wherein $\alpha_1$ = [GA-derived absorbance (D) of one of the GA certified reference materials measured in Step c)]/[GA concentration$_{[L]}$ (A) of the corresponding GA certified reference material determined in Step b)]).

Steps b), c), d), e$_0$), e), e$_1$), and f) can be performed as described above.

Step i) can be carried out, for example, by plotting the two or more GA concentrations$_{[L]}$ (A) as the vertical axis and two or more GA concentrations$_{[P]}$ [E] as the horizontal axis to set a linear regression equation, as shown later in Example.

By setting Regression equation IV as described above, the GA concentration$_{[L]}$ of the GA certified reference material can be correlated with the GA concentration$_{[P]}$ of the GA certified reference material measured by the enzymatic method and by using Regression equation IV and the measured albumin concentration, the certified GA value of the GA certified reference material can be correlated with the GA value of the GA certified reference material measured by the enzymatic method.

In the method of the present embodiment including the step of creating Regression equation IV, the above-described Step a), the above-described Steps a1) to a5), or the above-described Steps a1) to a3), Step C2, and Step C4 may be carried out prior to Step b).

The method of the present embodiment including the step of creating the above-described Regression equation IV may further include Steps B) and C):

B): a step of measuring a GA-derived absorbance (G) of a reference material and determining a GA concentration$_{[P]}$ (H) corresponding thereto according to Equation II$_0$, Equation II, or Equation II$_1$; and

C): a step of measuring an albumin-derived absorbance (I) of the reference material to determine an albumin concentration (J) of the reference material.

Steps B) and C) can be carried out as described above. By these steps, the reference material can be assigned with the concentration converted from the certified value.

The method of the present embodiment including the above-described step of creating Regression equation IV may further include the following Steps F1), F2), F5), and F6). It can be confirmed that by these steps, a certified value can be calculated by measuring and converting the value of the certified reference material based on a calibration curve prepared using the reference material.

F1) a step of determining, from the GA-derived absorbance (D) of the GA certified reference material, a GA concentration$_{[P]}$ (N) in the enzymatic method corresponding thereto by using a calibration curve prepared by using the reference material;

F2) a step of determining, from an albumin-derived absorbance (K) of the GA certified reference material used in Step F1), an albumin concentration (O) corresponding thereto by using a calibration curve prepared by using the reference material;

F5) a step of determining, from the GA concentration$_{[P]}$ (N), a GA concentration$_{[L]}$ (R) of the GA certified reference material according to Regression equation IV; and

F6) a step of determining a GA value ($Q_1'$) traceable to the certified value of the GA certified reference material used in Step F1) according to the following Equation 7:

$$\text{GA value } (Q_1') \text{ of GA certified reference material} = (\text{GA concentration}_{[L]}$$

$$(R))/(\text{albumin concentration (O)}) \quad \text{(Equation 7)}.$$

Steps F1) and F2) can be performed as described above.

In Step F5), the GA concentration$_{[L]}$ (R) of the GA certified reference material can be determined from the GA concentration$_{[P]}$ (N) obtained in Step F1) according to Regression equation IV and in Step F6), the GA value ($Q_1'$) of the GA certified reference material can be determined. In Step F6, unit adjustment may be performed as needed to obtain a GA value in the unit of mmol/mol. For example, when a GA concentration$_{[P]}$ ($\mu$mol/L) is divided by an albumin concentration ($\mu$mol/L), unit can be adjusted by multiplying by 1000.

Establishment of a traceability system of a GA value according to Regression equation IV can be confirmed by confirming that the GA value ($Q_1'$) obtained in Step F5) agrees with the certified value of the GA certified reference material used in Step F1). This means that a GA value traceable to a certified GA value can be measured conveniently by using Regression equation IV and the enzymatic method.

The above-described steps can be carried out in any order insofar as a measured value, regression equation, and the like necessary for each of the steps can be obtained. For example, Step b) and Step c) can be carried out in any order, Step B) and Step C) can be carried out in any order, and Step F1) and Step F2) can be carried out in any order.

In one aspect, the present embodiment also relates to a method of measuring a GA value traceable to a certified glycated-albumin (GA) value which method includes Step A$_3$). Step A$_3$) is a step of determining Regression equation V and it includes the following Steps b) to e$_0$) and j) to o):

b) a step of determining respective GA concentrations$_{[L]}$ (A) of two or more GA certified reference materials having respectively different certified GA values according to the following Equation 1:

$$\text{GA concentration}_{[L]} (A) = (\text{certified GA value (B) of GA certified}$$

$$\text{reference material}) \times (\text{albumin concentration (C) of GA certified reference}$$

$$\text{material}) \quad \text{(Equation 1)};$$

c) a step of measuring respective GA-derived absorbances (D) of the two or more GA certified reference materials;
d) a step of creating, from the two or more GA-derived absorbances (D) and the two or more GA concentrations$_{[L]}$ (A), the following Regression equation I:

$$D = \alpha A + \beta \quad \text{(Regression equation I);}$$

eo) a step of creating the following Linear equation $II_0$ having an intercept of zero:

$$D = \alpha E \quad \text{(Equation } II_0\text{)}$$

(wherein, $\alpha_0$ is an arbitrary positive value and E represents a GA concentration$_{[P]}$ (E) proportionate to the GA-derived absorbance (D)),

j) a step of measuring respective GA-derived absorbances (S) of two or more sample materials and determining GA concentrations$_{[L]}$ (T) corresponding thereto according to Regression equation I;

k) a step of measuring respective albumin-derived absorbances (U) of the two or more sample materials and determining albumin concentrations (V):

l) a step of determining, from the GA concentrations$_{[L]}$ (T), respective GA values$_1$ (W) of the two or more sample materials according to the following Equation 8:

$$\text{GA value}_1 \text{ (W)} = \text{(GA concentration}_{[L]} \text{ (T))/(albumin concentration (V))}$$

$$\text{(Equation 8);}$$

m) a step of determining, from the respective GA-derived absorbances (S) of the two or more sample materials, GA concentrations$_{[P]}$ (X) corresponding thereto according to Equation $II_0$;

n) a step of determining, from the GA concentrations$_{[P]}$ (X), respective GA values$_2$ (Y) of the two or more sample materials according to the following Equation 9:

$$\text{GA value}_2 \text{ (Y)} = \text{(GA concentration}_{[P]} \text{ (X))/(albumin concentration (V))}$$

$$\text{(Equation 9);}$$

and

o) a step of creating, from the GA values$_1$ (W) of the two or more sample materials and from the two or more GA values$_2$ (Y) corresponding thereto, the following Regression equation V:

$$W = \pi Y + \varphi \quad \text{(Regression equation V).}$$

In one aspect, the present embodiment also relates to a method of measuring a GA value traceable to a certified glycated-albumin (GA) value, wherein Equation $II_0$ in Step $e_0$) and Step m) included in Step $A_3$) is Equation II created by a step including the following Step e):

e) a step of creating Equation II by making the intercept of Regression equation I zero:

$$D = \alpha E \quad \text{(Equation II)}$$

(wherein E represents a GA concentration$_{[P]}$ (E) proportionate to the GA-derived absorbance (D)).

In one aspect, the present embodiment also relates to a method of measuring a GA value traceable to a certified glycated-albumin (GA) value, wherein Equation $II_0$ in Step $e_0$) and Step m) included in Step $A_3$) is Equation $II_1$ created by a step including the following Step $e_1$):

$e_1$) a step of creating the following Equation $II_1$ having an intercept of zero:

$$D = \alpha_1 E \quad \text{(Equation } II_1\text{)}$$

(wherein: $\alpha_1$ = [GA-derived absorbance (D) of one of the GA certified reference materials measured in Step c)]/[GA concentration$_{[L]}$ (A) of the corresponding GA certified reference material determined in Step b)]).

Steps b) to d), e$_0$), e), and e$_1$) can be performed as described above.

Steps j) and k) can be performed referring to Steps B) and C). In the present embodiment, the "sample material" is not particularly limited and any material can be used insofar as it contains glycated albumin. Preferred examples include a blood-derived component such as serum, plasma, and whole blood, and a product obtained by processing a blood component. Specific examples include healthy subject specimens, patient specimens, GAL control serum (Asahi Kasei Pharma), and GA-L calibrator (Asahi Kasei Pharma).

Steps l) to n) can be carried out in accordance with the procedure of each step while referring to Example described later. Step o) can be carried out, as shown later in Example, by plotting two or more GA values$_1$ (W) as the vertical axis and corresponding two or more GA values$_2$ (Y) as the horizontal axis, thereby setting a linear regression equation.

This method makes it possible to correlate, in Regression equation V, a GA value$_1$ (W) of an arbitrary sample material set via Regression equation I and a GA value$_2$ (Y) of the sample material obtained by the enzymatic method.

The above-described Steps f) and m) and Step B) can also be carried out using, instead of Equation II$_0$, Equation II' created by a method including the following steps:

a step of determining, from the GA-derived absorbance (S) of the sample material, a GA concentration$_{[L]}$ (T) corresponding thereto according to Regression equation I;

a step of creating Regression equation I' from the absorbance (S) and the GA concentration$_{[L]}$ (T); and

a step of creating Equation II' by making the intercept of Regression equation I' zero.

The above-described Steps f) and m) and Step B) can also be carried out using, instead of Equation II$_0$, Equation II" created by a method including the following steps:

a step of determining, from the GA-derived absorbance (S) of the sample material, a GA concentration$_{[L]}$ (T) corresponding thereto according to Regression equation I; and

a step of creating Equation II" having an intercept of zero and passing through two points, that is, the origin and a point set from the absorbance (S) and the GA concentration$_{[L]}$ (T) corresponding thereto.

The above-described Step j) can also be carried out using, instead of Regression equation I, Regression equation I' created by a method including the following steps:

a step of determining, from the GA-derived absorbance (S) of the sample material, a GA concentration$_{[L]}$ (T) corresponding thereto according to Regression equation I; and

a step of creating Regression equation I' from the absorbance (S) and the GA concentration$_{[L]}$ (T).

In the method of the present embodiment including the step of creating Regression equation V, the above-described Step a), Steps a1 to a5) or Steps a1) to a3) and Steps C2) and C4) may be performed prior to Step b).

The method of the present embodiment including the step of creating Regression equation V can further include the following Steps B) and C):

B) a step of measuring a GA-derived absorbance (G) of a reference material and determining a GA concentration$_{[P]}$ (H) corresponding thereto according to Equation II$_0$, Equation II, or Equation II$_1$; and

C) a step of measuring an albumin-derived absorbance (I) of the reference material and determining an albumin concentration (J) of the reference material.

Steps B) and C) can be carried out as described above. By these steps, the reference material can be assigned with a concentration converted from the certified value.

The method of the present embodiment including the step of creating Regression equation VI can further include the following Steps F1') to F4'). It can be confirmed that by these steps, a certified value can be calculated by measuring and converting the value of a certified reference material based on a calibration curve prepared using the reference material.

F1') a step of determining, from the GA-derived absorbance (S) of the sample material, a GA concentration$_{[P]}$ (N') corresponding thereto by using a calibration curve prepared by using the reference material;

F2') a step of determining, from the albumin-derived absorbance (U) of the sample material used in Step F1'), an albumin concentration (O') corresponding thereto by using a calibration curve prepared by using the reference material;

F3') a step of determining a GA value (P') according to the following Equation 6':

$$\text{GA value (P') of sample material} = (\text{GA concentration}_{[P]} \text{ (N')})/(\text{albumin}$$

$$\text{concentration (O')}) \quad \text{(Equation 6')};$$

and

F4') a step of determining, by using the GA value (P') and Regression equation V, a GA value (Q$_2$') traceable to a certified GA value$_1$ (W) with which the sample material used in Step F1') is assigned.

Steps F1') to F4') can be carried out referring to the above-described Steps F1) to F4).

Establishment of a traceability system of a GA value according to Regression equation V can be confirmed by confirming that the GA value (Q2') obtained in Step F4') agrees with the certified GA value$_1$ (W) with which the sample material used in Step F1') is assigned. This means that it is confirmed that a GA value traceable to a certified GA value can be measured conveniently by using Regression equation V and the enzymatic method.

The above-described steps can be carried out in any order insofar as a measured value, regression equation, and the like necessary for each of the steps can be obtained. For example, Steps b) and c) can be carried out in any order, Steps j) and k) can be carried out in any order, Steps B) and C) can be carried out in any order, and Steps F1') and F2') can be carried out in any order.

In one aspect, the present embodiment also relates to a method of measuring a GA value traceable to a certified glycated-albumin (GA) value, including Step A$_4$). Step A$_4$ is a step of determining Regression equation VI and it includes the following Steps b) to d), e$_0$), j), m), and p):

b) a step of determining respective GA concentrations$_{[L]}$ (A) of two or more GA certified reference materials having respectively different certified GA values according to the following Equation 1:

$$\text{GA concentration}_{[L]} \text{ (A)} = (\text{certified GA value (B) of GA certified}$$

$$\text{reference material}) \times (\text{albumin concentration (C) of GA certified reference}$$

$$\text{material}) \quad \text{(Equation 1)};$$

c) a step of measuring respective GA-derived absorbances (D) of two or more GA certified reference materials;

d) a step of creating, from the two or more GA-derived absorbances (D) and the two or more GA concentrations$_{[L]}$ (A), Regression equation I:

$$D = \alpha A + \beta \quad \text{(Regression equation I)};$$

e$_0$) a step of creating the following linear Equation II$_0$ having an intercept of zero:

$$D = \alpha_0 E \quad \text{(Equation II}_0\text{)}$$

(wherein: $\alpha_0$ is an arbitrary positive value; and E is a GA concentration$_{[P]}$ (E) proportionate to the GA-derived absorbance (D));

j) a step of measuring respective GA-derived absorbances (S) of two or more sample materials and determining GA concentrations$_{[L]}$ (T) corresponding thereto according to Regression equation I;

m) a step of determining, from the respective GA-derived absorbances (S) of the two or more sample materials, GA concentrations$_{[L]}$ (X) corresponding thereto according to Equation II$_0$;
p) a step of creating, from the respective GA concentrations$_{[L]}$ (T) and respective GA concentrations$_{[P]}$ (X) of the two or more sample materials, Regression equation VI:

$$T=\psi X+\omega \quad \text{(Regression equation VI).}$$

In one aspect, the present embodiment relates to a method of measuring a GA value traceable to a certified glycated-albumin (GA) value, wherein Equation II$_0$ in Step e$_0$) and Step m) included in Step A$_4$) is Equation II created by steps including the following Step e):
e) a step of creating Equation II by making the intercept of Regression equation I zero:

$$D=\alpha E \quad \text{(Equation II)}$$

(wherein E represents the GA concentration$_{[P]}$ (E) proportionate to the GA-derived absorbance (D))
In an aspect, the present embodiment relates to a method of measuring a GA value traceable to a certified glycated-albumin (GA) value, wherein Equation II$_0$ in Step e$_0$) and Step m) included in Step A$_4$) is Equation II$_1$ created by steps including the following Step e$_1$):
e$_1$) a step of creating Equation II$_1$ having an intercept of zero:

$$D=\alpha_1 E \quad \text{(Equation II}_1\text{)}$$

(wherein $\alpha_1$ = [GA-derived absorbance (D) of one of the GA certified reference materials measured in Step c)]/[GA concentration$_{[L]}$ (A) of the corresponding GA certified reference material determined in Step b)]).
Steps b) to d), e$_0$), e), e$_1$), j), and m) can be carried out as described above.
Step p) can be carried out, for example, by plotting the two or more GA concentrations$_{[L]}$ (T) as the vertical axis and the two or more GA concentrations$_{[P]}$ (X) as the horizontal axis and setting a linear regression equation, as shown later in Example.
This method makes it possible to determine a GA concentration$_{[L]}$ (T) of an arbitrary sample material through Regression equation I, determine a GA concentration$_{[P]}$ (X) of the arbitrary sample material through Equation II$_0$, and thereby correlate them according to Regression equation VI.
In the method of the present embodiment including the step of creating Regression equation VI, the above-described Step a); Steps a1) to a5); or Steps a1) to a3) and Steps C2) and C4) may be performed prior to Step b).
The method of the present embodiment including the step of creating Regression equation VI may further include Steps B) and C):

B) a step of measuring a GA-derived absorbance (G) of a reference material and determining a GA concentration$_{[P]}$ (H) corresponding thereto according to Equation II$_0$, Equation II, or Equation II$_1$; and
C) a step of measuring an albumin-derived absorbance (I) of the reference material and determining an albumin concentration (J) of the reference material.

Steps B) and C) can be carried out as described above. By these steps, the reference material can be assigned with the concentration converted from the certified value.
The method of the present embodiment including the step of creating Regression equation VI further includes, prior to Step b), the following Steps a1) to a3):

a1) a step of determining a molar certified albumin value (L) according to the following Equation 4:

$$\text{molar certified albumin value (L) = (certified albumin value of albumin}$$
$$\text{certified reference material)/(molecular weight of albumin)} \quad \text{(Equation 4);}$$

a2) a step of measuring an albumin-derived absorbance (M) of the albumin certified reference material; and

a3) a step of determining, from the molar certified albumin value (L) and the absorbance (M), $\theta$ that satisfies the following Equation 5:

$$M = \theta L \quad \text{(Equation 5)};$$

wherein Step $A_4$) further includes the following Step k):

k) a step of measuring respective albumin-derived absorbances (U) of two or more sample materials to determine an albumin concentration (V);

wherein Step $A_4$) further includes, after Steps j) and k), the following Step I):

l) a step of determining, from the GA concentrations$_{[L]}$ (T), respective GA values$_1$ (W) of two or more sample materials according to the following Equation 8:

$$\text{GA value}_1 \text{ (W)} = (\text{GA concentration}_{[L]} \text{ (T)})/(\text{albumin concentration (V)})$$

$$\text{(Equation 8)};$$

wherein the method further includes the following steps:

F1') a step of determining, from the GA-derived absorbance (S) of the sample material, a GA concentration$_{[P]}$ (N') in the enzymatic method corresponding thereto by using a calibration curve prepared by using the reference material;

F2') a step of determining, from the albumin-derived absorbance (U) of the sample material used in Step F1'), an albumin concentration (O') corresponding thereto by using a calibration curve prepared by using the reference material;

F5') a step of determining, from the GA concentration$_{[P]}$ (N'), a GA concentration$_{[L]}$ (R') of the sample material according to Regression equation VI; and

F6') a step of determining a GA value ($Q_3'$) traceable to a certified GA value$_1$ (W) set for the sample material used in Step F1') according to the following equation 7':

$$\text{GA value (Q}_3\text{') of sample material} = (\text{GA concentration}_{[L]} \text{ (R')})/(\text{albumin}$$

$$\text{concentration (O'))} \quad \text{(Equation 7')}.$$

Steps a1) to a3), k), and l) can be carried out as described above.

Steps F1'), F2'), F5'), and F6') can be carried out referring to Steps F1), F2), F5), and F6), respectively.

Establishment of a traceability system of a GA value according to Regression equation VI can be confirmed by confirming that the GA value ($Q_3'$) obtained in Step F6') agrees with the certified GA value$_1$ (W) set for the sample material used in Step F1'). This means that it is confirmed that a GA value traceable to a certified GA value can be measured conveniently by using Regression equation VI and the enzymatic method.

The above-described steps can be carried out in any order insofar as a measured value, regression equation, and the like necessary for each of the steps can be obtained. For example, Steps b) and c) can be carried out in any order, Steps B) and C) can be carried out in any order, and Steps F1') and F2') can be carried out in any order.

Thus, the present embodiment also relates to a method of measuring a GA value traceable to a certified GA value, including Step Ao of determining Regression equation III, Regression equation IV, Regression equation V, or Regression equation VI, that is, a step of determining:

a regression equation (Regression equation III) for converting a found GA value of a sample material to a certified GA value of a GA certified reference material,

a regression equation (Regression equation IV) for converting a found GA concentration$_{[P]}$ value of the sample material to a GA concentration$_{[L]}$ of the GA certified reference material,

a regression equation (Regression equation V) for converting a found GA value of the sample material to a GA value set for the sample material from the certified GA value of the GA certified reference material, or

a regression equation (Regression equation VI) for converting a found GA concentration$_{[P]}$ of the sample material to a GA concentration$_{[L]}$ set for the sample material from the GA concentration$_{[L]}$ of the GA certified reference material; and

Step Fo: a step of measuring a GA concentration$_{[P]}$ and an albumin concentration of the sample material by using a calibration curve prepared by using a reference material for which "a GA concentration$_{[P]}$ converted from the GA concentration$_{[L]}$ of the GA certified reference material" and "an albumin concentration converted from an albumin concentration of the GA certified reference material" are set and determining a GA value traceable to a certified GA value according to the regression equation determined in Step A$_0$). The term "GA concentration$_{[P]}$ converted from the GA concentration$_{[L]}$ of the GA certified reference material" has the same meaning as "GA concentration$_{[P]}$ associated with the GA concentration$_{[L]}$ of the GA certified reference material".

According to the method of the present embodiment, traceability of a GA value can be established. With respect to establishment of traceability in glycated hemoglobin A1c (HbA1c) which is a glycated protein similar to GA, traceability for the enzymatic method, which is a routine test method, from a value measured by a reference measurement procedure has already been established. This HbA1c is defined as "hemoglobin having glucose bound to the N-terminal valine of β chain thereof" and its glycation site is clearly known. Further, the reference measurement procedure of HbA1c is defined as a method of decomposing hemoglobin into glycated hexapeptide by using endoprotease Glu-C and thereby determining its quantity. The HbA1c enzymatic method, which is a routine test method, is a method of fragmenting glycated dipeptide (fructosyl-valine-histidine) at the N-terminal of a hemoglobin β chain by protease and measuring the resulting fragmented glycated dipeptide. It is therefore very easy to establish a traceability system for HbA1c because in HbA1c, there is a 1:1 relationship between an object of measurement for one molecule of hemoglobin in the reference measurement procedure and that of the enzymatic method which is a routine test.

On the other hand, GA is defined as "albumin having DOF-Lys" as described above and any site of 59 lysine residues present per molecule of albumin may be glycated. ID-MS, which is a reference measurement procedure of GA, performs measurement by fragmenting all of 59 lysine residues. The GA enzymatic method which is a routine test method uses protease. It is however difficult to fragment all the lysine residues by the protease treatment. In the case of GA, therefore, there is not a 1:1 relationship between an object of measurement for one molecule of albumin in the reference measurement procedure and that of the enzymatic method which is a routine test. Therefore, establishment of a traceability system of GA cannot be achieved in a manner similar to that of HbA1c.

By using various regression equations obtained by the method of the present embodiment, a GA value of a sample material traceable to a certified GA value can be measured conveniently. For example, the GA concentration$_{[P]}$ and the albumin concentration of a sample material are measured by the enzymatic method and based on these values, a GA value is determined.

From the GA value and Regression equation III or Regression equation V, a GA value of the sample material traceable to a certified GA value can be measured conveniently (In Regression equation V, the GA value is substituted for Y); or

from the GA concentration$_{[P]}$ and the Regression equation IV or the Regression equation VI, a GA concentration$_{[L]}$ can be determined and by dividing the GA concentration$_{[L]}$ by an albumin concentration, a GA value of the sample material traceable to a certified GA value can be measured conveniently.

In one aspect, the present embodiment also relates to a kit for measuring a GA value including a regression equation selected from the group consisting of Regression equation III, Regression equation IV, Regression equation V, and Regression equation VI;

a GA measurement reagent;

an albumin measurement reagent; and

a reference material. This measurement kit can measure the GA concentration$_{[P]}$ and albumin concentration of a sample material each by the enzymatic method and conveniently determine a GA value of the sample material traceable to a certified GA value. The reference material included in the kit is preferably a reference material whose GA concentration$_{[P]}$ is set according to Equation II$_0$, Equation II, or Equation II$_1$ by the above-described Step B) and Step C).

In one aspect, the present embodiment also relates to a computer program for identifying a GA value traceable to a certified GA value by ID-MS and for performing each step in the above-described measurement methods. For example, the program of the present embodiment may be a program which performs:

b) a step of determining respective GA concentrations$_{[L]}$ (A) of two or more GA certified reference materials having respectively different certified GA values according to the following Equation 1:

$$\text{GA concentration}_{[L]} \text{ (A)} = \text{(certified GA value (B) of GA certified}$$

$$\text{reference material)} \times \text{(albumin concentration (C) of GA certified reference}$$

$$\text{material)} \qquad \text{(Equation 1)}$$

c) a step of inputting respective GA-derived absorbances (D) of the two or more GA certified reference materials;
e$_0$) a step of creating the following Linear equation II$_0$ having an intercept of zero:

$$D = \alpha_0 E \qquad \text{(Equation II}_0\text{)};$$

(wherein: $\alpha_0$ is an arbitrary positive value; and E represents a GA concentration$_{[P]}$ (E) proportionate to the GA-derived absorbance (D)),
f) a step of determining two or more GA concentrations$_{[P]}$ (E) proportionate to the GA-derived absorbances (D) according to Equation II$_0$;
g) a step of determining two or more GA values (F) according to the following Equation 2:

$$\text{GA value (F)} = \text{(GA concentration}_{[P]} \text{ (E))/(albumin concentration (C))}$$

$$\text{(Equation 2)};$$

and
h) a step of creating the following Regression equation III:

$$B = \gamma F + \delta \qquad \text{(Regression equation III)}$$

from the respective certified GA values (B) of the two or more GA certified reference materials and the two or more GA values (F) corresponding thereto.

In one aspect, the above-described program may be a program in which Step e$_0$) includes the following Step d) and Step e):

d) a step of creating the following Regression equation I:

$$D = \alpha A + \beta \qquad \text{(Regression equation I)}$$

from the two or more GA-derived absorbances (D) and the two or more GA concentrations$_{[L]}$ (A); and
e) a step of creating the following Equation II:

$$D = \alpha E \qquad \text{(Equation II)}$$

by making the intercept of Regression equation I zero, and
Equation II$_0$ in Step f) is Equation II.

In one aspect, the above-described program may be a program wherein:
Step e$_0$) is Step e$_1$) shown below:
e$_1$) a step of creating the following Equation II$_1$ having as an intercept thereof zero:

$$D = \alpha_1 E \quad \text{(Equation II}_1\text{)}$$

(wherein $\alpha_1$ = [GA-derived absorbance (D) of one of the GA certified reference materials measured in Step c)]/[GA concentration$_{[L]}$ (A) of the corresponding GA certified reference material determined in Step b)]); and Equation II$_0$ in Step f) is Equation II$_1$.

Similarly, the program of the present embodiment may be a program for performing steps in another method of the present embodiment.

In one aspect, the present embodiment relates to an apparatus for measuring a GA value traceable to a certified GA value determined by ID-MS, wherein the apparatus is equipped with units for performing steps in each of the above-described measurement methods. For example, the apparatus of the present embodiment may be equipped with:

b) a unit for determining respective GA concentrations$_{[L]}$ (A) of two or more GA certified reference materials having respectively different certified GA values according to the following Equation 1:

$$\text{GA concentration}_{[L]} \text{ (A)} = \text{(certified GA value (B) of GA certified reference material)} \times \text{(albumin concentration (C) of GA certified reference material)} \quad \text{(Equation 1)};$$

c) a unit for measuring respective GA-derived absorbances (D) of the two or more GA certified reference materials;
e$_0$) a unit for creating the following Linear equation II$_0$ having an intercept of zero:

$$D = \alpha_0 E \quad \text{(Equation II}_0\text{)};$$

(wherein: $\alpha_0$ is an arbitrary positive value; and E represents a GA concentration$_{[P]}$ (E) proportionate to the GA-derived absorbance (D)),
f) a unit for determining two or more GA concentrations$_{[P]}$ (E) proportionate to the GA-derived absorbances (D) according to Equation II$_0$;
g) a unit for determining two or more GA values (F) according to the following Equation 2:

$$\text{GA value (F)} = \text{(GA concentration}_{[P]} \text{ (E))/(albumin concentration (C))} \quad \text{(Equation 2)};$$

and
h) a unit for creating Regression equation III:

$$B = \gamma F + \delta \quad \text{(Regression equation III)}$$

from the certified GA values (B) of the two or more GA certified reference materials and the two or more GA values (F) corresponding thereto.

In one aspect, the above-described apparatus may be an apparatus wherein:
Unit e$_0$) includes the following Unit d) and Unit e):
d) a unit for creating, from the two or more GA-derived absorbances (D) and the two or more GA concentrations$_{[L]}$ (A), Regression equation (I):

$$D = \alpha A + \beta \quad \text{(Regression equation I)}$$

and

e) a unit for creating Equation (II) by making the intercept of Regression equation I zero:

$$D = \alpha E \quad \text{(Equation II)};$$

and
Equation $II_0$ in Unit f) is Equation II.

In another aspect, the apparatus may be an apparatus, wherein:
Unit $e_0$) is the following Unit $e_1$):
$e_1$) a unit for creating the following Equation $II_1$ having an intercept of zero:

$$D = \alpha_1 E \quad \text{(Equation II}_1\text{)}$$

(wherein $\alpha_1$ = [GA-derived absorbance (D) of one of the GA certified reference materials measured in Unit c)]/[GA concentration$_{[L]}$ (A) of the corresponding GA certified reference material determined in Unit b)]); and
Equation $II_0$ in Unit f) is Equation $II_1$.
Similarly, the apparatus of the present embodiment may be an apparatus equipped with a unit for performing steps in another method of the present embodiment.
In one aspect, the present embodiment relates to an apparatus for measuring a GA value traceable to a certified glycated-albumin (GA) value, including:

an operation unit for which a equation selected from the group consisting of Equation III, Equation IV, Equation V, and Equation VI is set;
a calibration curve preparation unit for preparing a calibration curve by using a reference material having a GA concentration$_{[P]}$ set therefor according to Equation $II_0$, Equation II, or Equation $II_1$, or a calibration curve preparation unit in which Equation $II_0$, Equation II, or Equation $II_1$ is incorporated in operation; and
a measuring unit for measuring a GA value.

Here, each equation can be determined using the above-described arbitrary method.
In one aspect, the present embodiment also relates to a method of measuring a GA value traceable to a certified glycated-albumin (GA) value, including the following 1) reference material and 2) operation program:

1) a reference material whose GA concentration$_{[P]}$ is set according to Equation $II_0$) created in Step $e_0$), Equation II created in Step e), or Equation $II_1$ created in Step $e_1$); and

2) an operation program having, incorporated therein, a equation selected from the group consisting of Equation III, Equation IV, Equation V, and Equation VI.

Each Equation in 2) can be determined by the above-described arbitrary method.

Examples

Next, the present embodiment will be described more specifically by Examples and Comparative Examples (Examples and the like), but the present invention is not limited to or by them. In the following Examples and the like, a glycated albumin concentration$_{[L]}$ is a concentration of glycated lysine residues (DOF-Lys) and a glycated albumin concentration$_{[P]}$ is a concentration of a glycated amino acid or glycated peptide.
The following are used in Examples 1 to 11 and Comparative Example 1.

1) Glycated albumin certified reference material

Certified Reference Material for Measurement of Glycated Albumin in Human Serum (JCCRM 611-1) produced and sold by Reference Material Institute for Clinical Chemistry Standards was used. It is provided at three concentration levels, M, H, and HH in ascending order of a certified value (glycated albumin value).

2) Albumin certified reference material

IFCC plasma protein international reference material (ERM-DA 470k/IFCC) sold by Reference Material Institute for Clinical Chemistry Standards was used.

3) Measurement reagent

Lucica GA-L (Asahi Kasei Pharma) was used as a reagent for measuring the absorbance of albumin (ALB) (modified BCP method) and glycated albumin.
Lucica GA-L for a glycated albumin reagent is composed of a first reagent (pre-treatment solution) and a second reagent (enzyme solution) and that for an ALB reagent is composed of a first reagent (pre-treatment solution) and a second reagent (color developer solution).

4) Reference material

As a reference material, a GA-L calibrator (Asahi Kasei Pharma) was used as a high reference material.

5) Measurement apparatus

As an apparatus for measurement of absorbance and conversion from absorbance to concentration, a Hitachi 7180 Automatic analyzer (Hitachi High-Tech Fielding) was used.

6) Glycated albumin measurement method

By using the measurement apparatus described above in 5), measurement was performed in a 10-min mode with 4.5 $\mu$L of a sample, 180 $\mu$L of a first reagent, and 45 $\mu$L of a second reagent. At photometry point 0, sampling and stirring of the sample and the first reagent were performed, measurement was started, and a first reaction was carried out. Then, after sampling and stirring of the second reagent at point 16, a second reaction was carried out. Measurement was performed until 34 points in total.
The other operation was performed based on the package insert or instruction manual.

7) Calculation method of glycated albumin absorbance

The glycated albumin absorbance (change in measured absorbance of measurement sample) was determined according to the following calculation equation, while using an average absorbance between points 33 and 34 and an average absorbance between points 15 and 16 before addition of the second reagent.

$$\Delta mAbs = (Ab1-(Ab2\times0.8))/10$$

$\Delta mAbs$: change in absorbance of glycated albumin

Ab1: average absorbance between points 33 and 34

Ab2: average absorbance between points 15 and 16

In the above equation, "0.8" is a value calculated according to the following calculation equation:

$$\text{(amount of first reagent (180 µL))/(amount of first reagent (180 µL)} +$$

$$\text{amount of second reagent (45 µL)) = 0.8}$$

8) Albumin measurement method

By using the measurement apparatus described above in 5), measurement was performed in a 10-min mode with 3.0 $\mu$L of a sample, 220 $\mu$L of the first reagent, and 110 $\mu$L of the second reagent. At 0 point, sampling and stirring of the sample and the first reagent were performed, measurement was started, and a first reaction was carried out. Then, after sampling and stirring of the second reagent at point 16, a second reaction was carried out. Measurement was performed until 34 points in total.

The other operation was performed based on the package insert or instruction manual.

9) Calculation method of albumin (ALB) absorbance

An ALB absorbance (change in measured absorbance of measurement sample) was determined according to the following calculation equation, while using an average absorbance between points 33 and 34 and an average absorbance between points 13 and 14 before addition of the second reagent.

$$\Delta mAbs = (Ab1-(Ab2\times0.67))/10$$

$\Delta mAbs$: change in ALB absorbance

Ab1: average absorbance between points 33 and 34

Ab2: average absorbance between points 13 and 14

In the above equation, "0.67" is a value calculated according to the following calculation equation:

Amount of first reagent (220 μL)/(amount of first reagent (220 μL) +

amount of second reagent (110 μL)) = 0.67.

[Example 1]

<Determination of albumin concentration of glycated albumin certified reference material>

1) The albumin-derived absorbance (M) of the albumin certified reference material and the albumin-derived absorbance (K) of the glycated albumin certified reference materials were measured using Lucica GA-L.

2) The certified albumin value (3.72 g/dL) of the albumin certified reference material was converted into molar unit. The molecular weight used for conversion was 66438 (Geisow MJ et. Al. In: Villafranca JJ, ed. Techniques in proteinchemistry. San Diego, CA: Academic Press, 1991: 576-572). As the result, a molar certified albumin value (L) was 559.9 $\mu$mol/L.

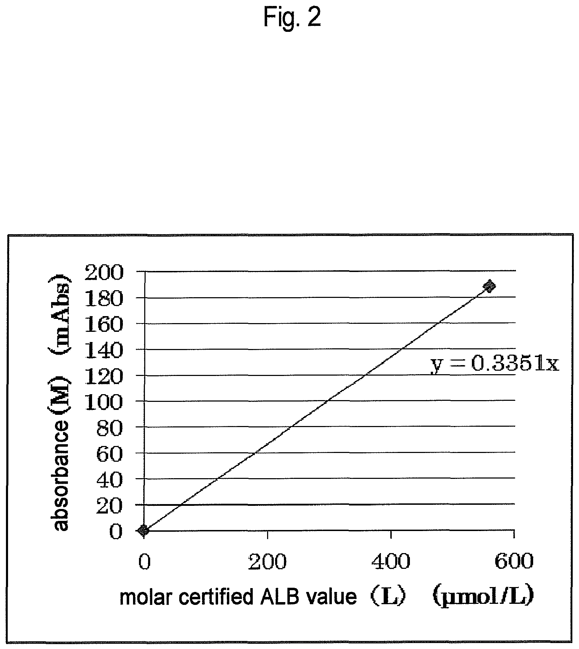

3) A regression equation (Equation 5): y=0.3351x was created by plotting the molar certified albumin value (L) obtained by the unit conversion as the horizontal axis and the albumin-derived absorbance (M) as the vertical axis (FIG. 2).

4) The albumin absorbance (K) of the glycated albumin certified reference materials was substituted in Linear regression equation (Equation 5) to determine an albumin concentration (C) of the glycated albumin certified reference materials.

<Determination of glycated albumin concentration$_{[L]}$ of glycated albumin certified reference material>

1) The glycated albumin certified reference materials had the following certified glycated albumin values (B) in ascending order: M: 230, H: 365, and HH: 564 mmol/mol. A glycated albumin concentration$_{[L]}$ (A) was determined by multiplying each of the certified glycated-albumin values by the albumin concentration (C) determined above.

<Determination of linear regression equation for associating the glycated albumin absorbance derived from the glycated albumin certified reference materials with the glycated albumin concentration$_{[L]}$>
1) Lucica GA-L was used to measure a glycated albumin-derived absorbance (D) of the glycated albumin certified reference materials.

The values determined as described above are listed in Table 1.

[Table 1]

| Item | Glycated albumin certified reference material | Albumin certified reference material |
|---|---|---|
| Certified glycated-albumin value (glycated albumin value) (mmol/mol) | B: M 230, H 365, HH 564 | - |
| Certified albumin value (albumin concentration) (g/dL) | - | 3.72 g/dL |
| Molar certified albumin value (albumin concentration) ($\mu$mol/L) | - | L: 559.9 $\mu$mol/L |
| Albumin-derived absorbance (mAbs) | K: M 217.9, H 208.5, HH 196.0 | M: 187.6 |
| Albumin concentration ($\mu$mol/L) | C: M 650.3, H 622.2, HH 585.0 | - |
| Glycated albumin-derived absorbance (mAbs) | D: M 27.1, H 45.4, HH 69.4 | - |
| Glycated albumin concentration$_{[L]}$ ($\mu$mol/L) | A: M 149.6, H 227.1, HH 330.0 | - |

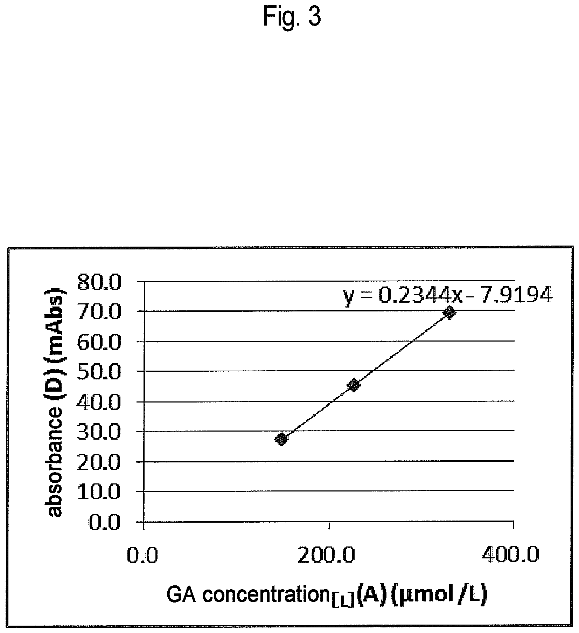

2. By plotting the glycated albumin concentration$_{[L]}$ (A) of the glycated albumin certified reference materials as the horizontal axis and the glycated albumin-derived absorbance (D) as the vertical axis, Linear regression equation (I): $y=0.2344x - 7.9194$ was set (FIG. 3).

[Example 2]

To make the measured value agree with the certified value, setting of two-stage linear regression equation was investigated.
<Setting of Linear equation (II) by making the intercept of Linear regression equation (I) zero>

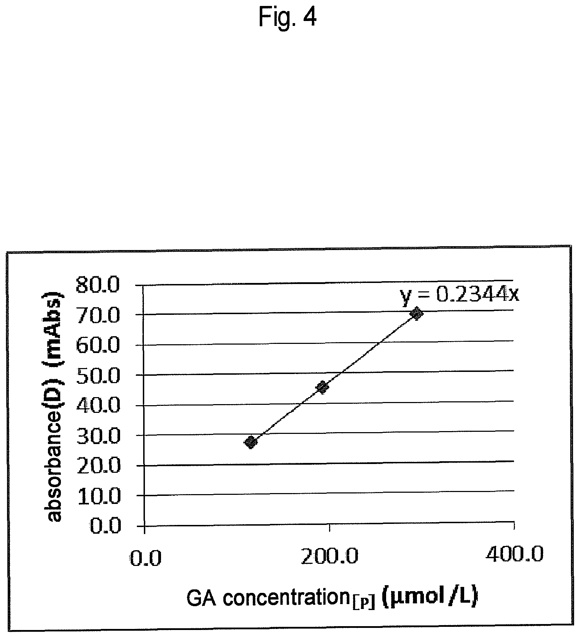

1) Linear regression equation (I) was moved in parallel to make its intercept 0 and Linear equation (II): $y=0.2344x$ was set (FIG. 4).

<Setting of Linear regression equation (III) for conversion of measured value into certified value>
1) A glycated albumin concentration$_{[P]}$ (E) was calculated by substituting the glycated albumin-derived absorbance (D) in Linear equation (II) obtained above.
2) A glycated albumin value (F) was calculated by dividing the glycated albumin concentration$_{[P]}$ (E) by the albumin concentration (C) and multiplying the quotient by 1000.
Values of the glycated albumin certified reference materials obtained according to Linear equation (II) are listed in Table 2.

[Table 2]

| Item | Glycated albumin certified reference material |
|---|---|
| Certified glycated-albumin value (glycated albumin value) (mmol/mol)) | B: M 230, H 365, HH 564 |
| Glycated albumin concentration$_{[P]}$ ($\mu$mol/L) | E: M 115.6, H 193.7, HH 296.1 |
| Albumin concentration ($\mu$mol/L) | C: M 650.3, H 622.2, HH 585.0 |
| Glycated albumin value, measured value (mmol/mol) | F: M 178, H 311, HH 506 |

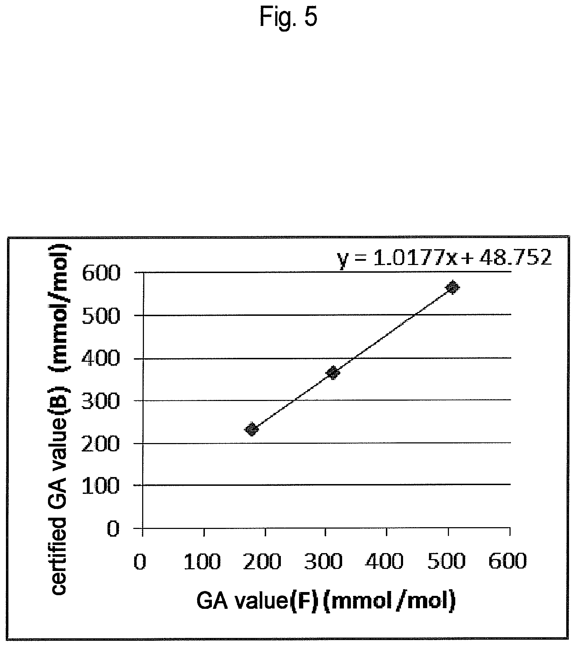

3) The certified glycated-albumin value (B) of the glycated albumin certified reference materials was plotted as the vertical axis and the glycated albumin value (F) was plotted as the horizontal axis and Linear regression equation (III): $y=1.0177x + 48.752$ was set (FIG. 5).

[Example 3]

<Setting of values for reference material according to Linear equation (II)>

1) A glycated albumin concentration$_{[L]}$ (H) of the reference material was calculated by measuring a glycated albumin-derived absorbance (G) of the reference material and substituting it in Linear equation (II).

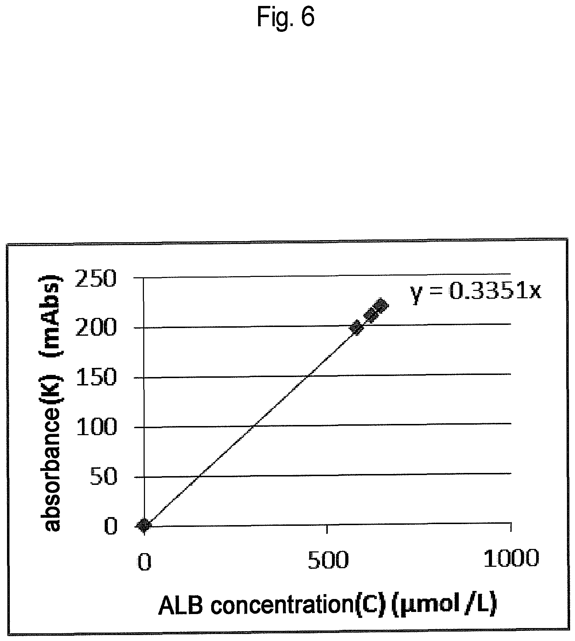

2) Linear regression equation (Equation 3): y=0.3351x was set by plotting the albumin concentration (C) of the glycated albumin certified reference materials as the horizontal axis and the albumin-derived absorbance (K) of the glycated albumin certified reference materials as the vertical axis (FIG. 6).

3) An albumin concentration (J) of the reference material was calculated by measuring an albumin-derived absorbance (I) of the reference material and substituting it in Linear regression equation (Equation 3).

Values of the reference material according to Linear equation (II) are listed in Table 3.

[Table 3]

| Item | Reference material |
|---|---|
| Albumin-derived absorbance (mAbs) | I: 257.8 |
| Albumin concentration (μmol/L) | J: 769.3 |
| Glycated albumin-derived absorbance (mAbs) | G: 88.6 |
| Glycated albumin concentration$_{[P]}$ (μmol/L) | H: 378.0 |

[Example 4]

<Step of obtaining, from measured values calculated using the reference material whose glycated albumin concentration and albumin concentration were determined according to Linear equation (II), a value traceable to the certified value of the glycated albumin certified reference materials according to Linear regression equation (III)>

1) A glycated albumin concentration$_{[P]}$ [N] of the glycated albumin certified reference materials was measured by preparing a calibration curve using the reference material whose values were set in Example 3.

2) An albumin concentration [O] of the glycated albumin certified reference materials was measured by preparing a calibration curve using the reference material whose values were set in Example 3.

3) A glycated albumin value (P) was calculated by dividing the glycated albumin concentration$_{[P]}$ (N) of the glycated albumin certified reference materials by the albumin concentration (O) and multiplying the quotient by 1000.

4) A glycated albumin value (Q) was obtained by substituting the glycated albumin value (P) in Linear regression equation (III).

Values obtained by converting, according to Linear regression equation (III), the measured values of the glycated albumin certified reference materials calculated according to Linear equation (II) are listed in Table 4.

[Table 4]

| Item | Glycated albumin certified reference material |
|---|---|
| Certified glycated-albumin value (glycated albumin value) (mmol/mol) | B: M 230, H 365, HH 564 |
| Glycated albumin concentration$_{[P]}$ (μmol/L) | N: M 115.6, H 193.7, HH 296.1 |
| Albumin concentration (μmol/L) | O: M 650.3, H 622.2, HH 585.0 |
| Glycated albumin value, measured value (mmol/mol) | P: M 178, H 311, HH 506 |

(continued)

| Item | Glycated albumin certified reference material |
|---|---|
| Glycated albumin value, converted value (mmol/mol) | Q: M 230, H 366, HH 564 |

As is apparent from Table 4, the converted value (Q) obtained by using the reference material whose values were set according to Linear equation (II) and converting the measured value (P) of the glycated albumin certified reference material according to Linear regression equation (III) agreed well with the certified value (B) specified for the glycated albumin certified reference material. This means that a series of sequential traceability system can be established, judging from that the certified value of the glycated albumin certified reference material is obtained by calculation using the reference material whose values are set with the glycated albumin certified reference material as an upper level standard.

[Example 5]

<Setting of albumin concentration of reference material by using calibration curve of albumin certified reference material>

1) An albumin concentration (J') of the reference material was calculated by measuring an albumin-derived absorbance (I) of the reference material and substituting it in Linear regression equation (Equation 5).

Value of the reference material obtained using a calibration curve of the albumin certified reference material is described in Table 5.

[Table 5]

| Item | Reference material |
|---|---|
| Albumin concentration ($\mu$mol/L) | J': 769.3 |

Since the albumin concentration (J') of the reference material obtained by substituting the albumin-derived absorbance (I) in Linear regression equation (Equation 5) agreed with the albumin concentration (J), it has been confirmed that an albumin concentration of the reference material can also be found according to Linear regression equation (Equation 5).

It has been confirmed that a traceability system can be established as in Example 4 except that instead of the albumin concentration (J), the albumin concentration (J') is set for the reference material.

[Example 6]

<Setting of Linear equation ($II_{(d)}$) by making the intercept of Linear regression equation (I) 0>

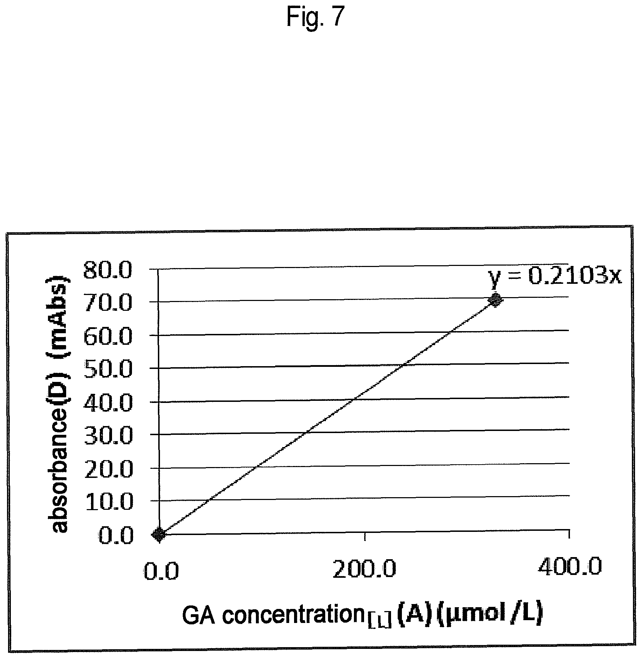

1) Linear equation ($II_{(d)}$): y=0.2103x was set by changing the gradient of Linear regression equation (I) to make the intercept 0 based on the glycated albumin concentration$_{[L]}$ (A) 330.0 $\mu$mol/L of the glycated albumin certified reference material HH obtained in Example 1 (FIG. 7).

<Setting of Linear regression equation ($III_{(d)}$) for conversion of measured value to certified value>
1) A glycated albumin concentration$_{[P]}$ ($E_{(d)}$) was calculated by substituting the glycated albumin-derived absorbance (D) in Linear equation ($II_{(d)}$).
2) A glycated albumin value ($F_{(d)}$) was calculated by dividing the glycated albumin concentration$_{[P]}$ ($E_{(d)}$) by the albumin concentration (C) and then, multiplying the quotient by 1000.
Values of the glycated albumin certified reference materials according to the linear equation ($II_{(d)}$) are listed in Table 6.

[Table 6]

| Item | Glycated albumin certified reference material |
|---|---|
| Certified glycated-albumin value (glycated albumin value) (mmol/mol)) | B: M 230, H 365, HH 564 |

(continued)

| Item | Glycated albumin certified reference material |
| --- | --- |
| Glycated albumin concentration$_{[P]}$ ($\mu$mol/L) | $E_{(d)}$: M 128.9, H 215.9, HH 330.0 |
| Albumin concentration ($\mu$mol/L) | C: M 650.3, H 622.2, HH 585.0 |
| Glycated albumin value, measured value (mmol/mol) | $F_{(d)}$: M 198, H 347, HH 564 |

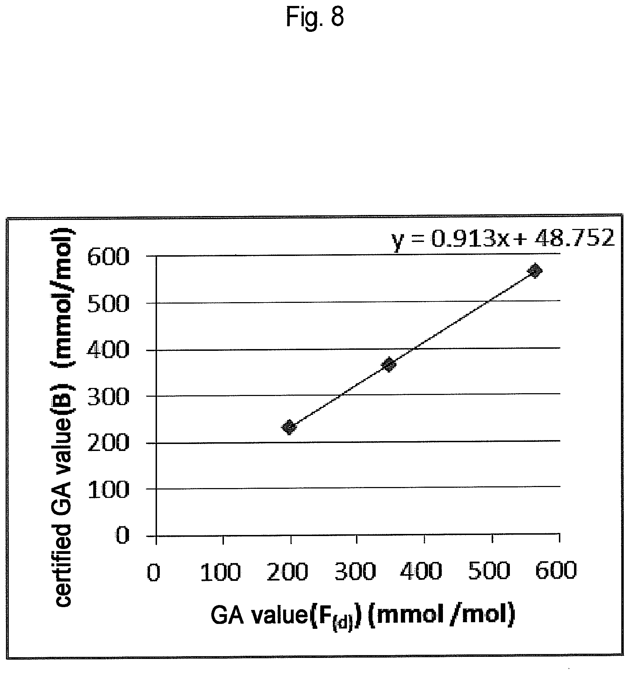

3) Linear regression equation ($III_{(d)}$): y=0.913x + 48.752 was set by plotting the certified glycated-albumin value (B) of the glycated albumin certified reference material as the vertical axis and the glycated albumin value ($F_{(d)}$) as the horizontal axis (FIG. 8).

[Example 7]

<Setting of values for reference material according to Linear equation ($II_{(d)}$)>

1) A glycated albumin concentration$_{[P]}$ ($H_{(d)}$) of the reference material was calculated by measuring a glycated albumin-derived absorbance (G) of the reference material and then substituting it in Linear equation ($II_{(d)}$).

2) An albumin concentration (J) of the reference material was calculated using the method described in Example 3.

Values of the reference material according to Linear equation ($II_{(d)}$) are listed in Table 7.

[Table 7]

| Item | Reference material |
| --- | --- |
| Albumin concentration ($\mu$mol/L) | J: 769.3 |
| Glycated albumin concentration$_{[P]}$ ($\mu$mol/L) | $H_{(d)}$: 421.3 |

[Example 8]

< Step of obtaining, from measured values calculated using the reference material whose glycated albumin concentration and albumin concentration were determined according to Linear equation ($II_{(d)}$), a value traceable to the certified value of the glycated albumin certified reference materials according to Linear regression equation ($III_{(d)}$)>

1) A glycated albumin concentration$_{[P]}$ [$N_{(d)}$] of the glycated albumin certified reference material was measured by preparing a calibration curve using the reference material whose values were set in Example 7.

2) A glycated albumin value ($P_{(d)}$) was calculated by dividing the glycated albumin concentration$_{[P]}$ ($N_{(d)}$) of the glycated albumin certified reference material by the albumin concentration (O) obtained in Example 4 and multiplying the quotient by 1000.

4) A glycated albumin value ($Q_{(d)}$) was obtained by substituting the glycated albumin value ($P_{(d)}$) in Linear regression equation ($III_{(d)}$).

Values obtained by converting, according to Linear regression equation ($III_{(d)}$), the measured values of the glycated albumin certified reference materials calculated according to Linear equation ($II_{(d)}$) are listed in Table 8.

[Table 8]

| Item | Glycated albumin certified reference material |
| --- | --- |
| Certified glycated-albumin value (glycated albumin value) (mmol/mol) | B: M 230, H 365, HH 564 |
| Glycated albumin concentration$_{[P]}$ ($\mu$mol/L) | $N_{(d)}$: M 128.9, H 215.9, HH 330.0 |

(continued)

| Item | Glycated albumin certified reference material |
|------|-----------------------------------------------|
| Albumin concentration ($\mu$mol/L) | O: M 650.3, H 622.2, HH 585.0 |
| Glycated albumin value, measured value (mmol/mol) | $P_{(d)}$: M 198, H 347, HH 564 |
| Glycated albumin value, converted value (mmol/mol) | $Q_{(d)}$: M 230, H 366, HH 564 |

As is apparent from Table 8, the converted value ($Q_{(d)}$) obtained by using the reference material whose values were set according to Linear equation ($II_{(d)}$) and converting the measured value ($P_{(d)}$) of the glycated albumin certified reference material according to Linear regression equation ($III_{(d)}$) agreed well with the certified value (B) specified for the glycated albumin certified reference material. This means that a series of sequential traceability system can be established, judging from that the certified value of the glycated albumin certified reference material is obtained by calculation using the reference material whose values are set with the glycated albumin certified reference material as an upper level standard.

[Example 9]

Establishment of a traceability system by setting a regression equation while using the glycated albumin concentration of the glycated albumin certified reference material was investigated.

<Setting of Linear regression equation (IV)>

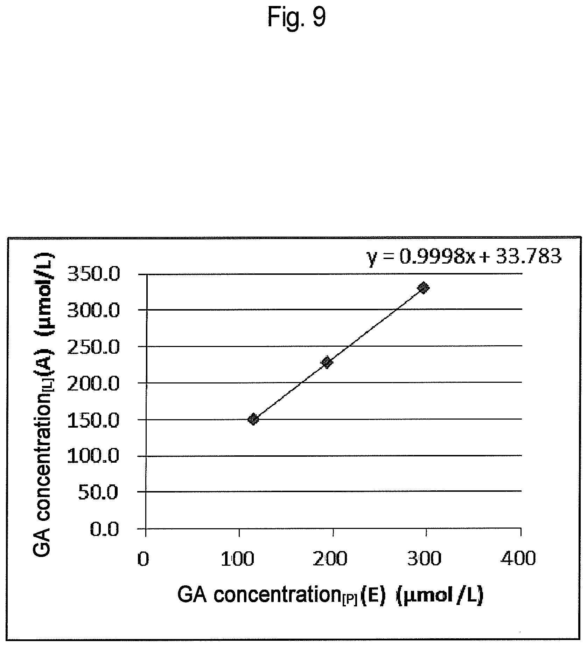

1) By plotting the glycated albumin concentration$_{[L]}$ (A) of the glycated albumin certified reference materials as the vertical axis and the glycated albumin concentration$_{[P]}$ (E) as the horizontal axis, Linear regression equation (IV): y=0.9998x + 33.783 was set (FIG. 9).

<Step of obtaining a traceable value by converting, according to Linear regression equation (IV), a measured value calculated using the reference material whose values were set according to Linear equation (II) >

1) A glycated albumin concentration$_{[P]}$ (N) was measured by preparing a calibration curve using the reference material whose values were set according to Linear equation (II) in Example 3 and using the glycated albumin-derived absorbance (D) of the glycated albumin certified reference material.

2) A glycated albumin concentration$_{[L]}$ (R) was obtained by substituting the glycated albumin concentration$_{[P]}$ (N) in Linear regression equation (IV).

3) A glycated albumin value ($Q_{1'}$) was calculated by dividing the glycated albumin concentration$_{[L]}$ (R) of the glycated albumin certified reference material by the albumin concentration (O) and multiplying the quotient by 1000.

Values obtained by converting, according to Linear regression equation (IV), the measured values of the glycated albumin certified reference materials calculated according to Linear equation (II) are listed in Table 9.

[Table 9]

| Item | Glycated albumin certified reference material |
|------|-----------------------------------------------|
| Certified glycated-albumin value (glycated albumin value) (mmol/mol) | B: M 230, H 365, HH 564 |
| Glycated albumin concentration$_{[P]}$ ($\mu$mol/L) | N: M 115.6, H 193.7, HH 296.1 |
| Glycated albumin concentration$_{[L]}$ ($\mu$mol/L) | R: M 149.4, H 227.4, HH 329.8 |
| Albumin concentration ($\mu$mol/L) | O: M 650.3, H 622.2, HH 585.0 |
| Glycated albumin value (mmol/mol) | $Q_1'$: M 230, H 366, HH 564 |

As is apparent from Table 9, the glycated albumin value (Q$_1$') obtained by using the reference material whose values were set according to Linear equation (II) and dividing, by the albumin concentration (O), the glycated albumin concentration$_{[L]}$ (R) obtained by converting the glycated albumin concentration$_{[P]}$ (N) of the glycated albumin certified reference material according to Linear regression equation (IV) agreed well with the certified value (B) specified for the glycated albumin certified reference material. This means that a series of sequential traceability system can be established, judging from that the certified value of the glycated albumin certified reference material is obtained by calculation using the reference material whose values are set with the glycated albumin certified reference material as an upper level standard.

In Examples 10 and 11, the following materials were additionally used.

1) Sample material

GA-L control serums L and H (Asahi Kasei Pharma) are used.

The control serums are comprised of L and H in ascending order of concentration.

[Example 10]

Establishment of a traceability system by setting a regression equation using a sample material was investigated.

<Setting of various values for sample material>

1) A glycated albumin concentration$_{[L]}$ (T) of a sample material was determined by measuring a glycated albumin-derived absorbance (S) of the sample material and substituting it in Linear regression equation (I).

2) An albumin concentration (V) of the sample material was determined by measuring an albumin-derived absorbance (U) of the sample material and substituting it in Linear regression equation (Equation 5).

3) A glycated albumin value (W) (set value) traceable from the glycated albumin certified reference material was calculated by dividing the glycated albumin concentration$_{[L]}$ (T) by the albumin concentration (V) and multiplying the quotient by 1000.

<Setting of Linear regression equation (V)>

1) A glycated albumin concentration$_{[P]}$ (X) of the sample material was calculated by substituting the glycated albumin-derived absorbance (S) of the sample material in Linear equation (II).

2) A glycated albumin value (Y) was calculated by dividing the glycated albumin concentration$_{[P]}$ (X) by the albumin concentration (V) and then, multiplying the quotient by 1000.

Values of the sample material are listed in Table 10.

[Table 10]

| Item | Sample material |
| --- | --- |
| Albumin-derived absorbance (mAbs) | U: L 242.8, H 250.1 |
| Albumin concentration ($\mu$mol/L) | V: L 724.6, H 746.3 |
| Glycated albumin-derived absorbance (mAbs) | S: L 36.6, H 99.0 |
| Glycated albumin concentration$_{[L]}$ ($\mu$mol/L) | T: L 189.9, H 456.1 |
| Glycated albumin value, set value (mmol/mol) | W: L 262, H 611 |
| Glycated albumin concentration$_{[P]}$ ($\mu$mol/L) | X: L 156.1, H 422.4 |
| Glycated albumin value (mmol/mol) | Y: L 216, H 566 |

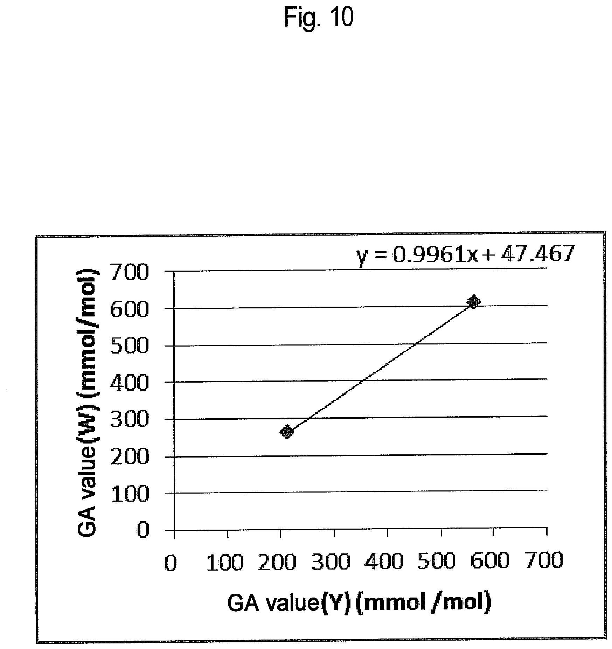

Regression equation (V): y=0.9961x + 47.467 was set by plotting the glycated albumin value (W) (set value) as the vertical axis and the glycated albumin value (Y) as the horizontal axis (FIG. 10).

<Step of obtaining a traceable value by using, according to Linear regression equation (V), a measured value calculated using a reference material whose values are set according to Linear equation (II)>

1) A glycated albumin concentration$_{[P]}$ [N'] of a sample material was measured by preparing a calibration curve using the reference material whose values were set according to Linear equation (II) in Example 3.

2) An albumin concentration (O') of the sample material was measured by preparing a calibration curve using the reference material whose values were set according to Linear regression equation (Equation 5) in Example 5.

3) A glycated albumin value (P') was calculated by dividing the glycated albumin concentration$_{[P]}$ (N') of the sample material by the albumin concentration (O') and multiplying the quotient by 1000.

4) A glycated albumin value (Q$_2$') was obtained by substituting the glycated albumin value (P') in Linear regression equation (V).

Measured values of the sample material according to Linear equation (II) and converted values according to Linear regression equation (V) are listed in Table 11.

[Table 11]

| Item | Sample material |
| --- | --- |
| Glycated albumin value, set value (mmol/mol) | W: L 262, H 611 |
| Glycated albumin concentration$_{[P]}$ ($\mu$mol/L) | N': L 156.1, H 422.4 |
| Albumin concentration ($\mu$mol/L) | O': L 724.6, H 746.3 |
| Glycated albumin value, measured value (mmol/mol) | P': L 216, H 566 |
| Glycated albumin value, converted value (mmol/mol) | Q$_2$': L 262, H 611 |

As is apparent from Table 11, the converted value (Q2') obtained by using the reference material whose values were set according to Linear equation (II) and converting the glycated albumin value (P') of the sample material according to Linear regression equation (V) agreed well with the glycated albumin value (W) set for the sample material. This means that a series of sequential traceability system can be established, judging from that a set value of the sample material set from the glycated albumin certified reference material is calculated by using the reference material whose values are set with the glycated albumin certified reference material as an upper level standard.

[Example 11]

Establishment of a traceability system by setting a regression equation using a glycated albumin concentration of a sample material was investigated.

<Setting of Linear regression equation (VI)>

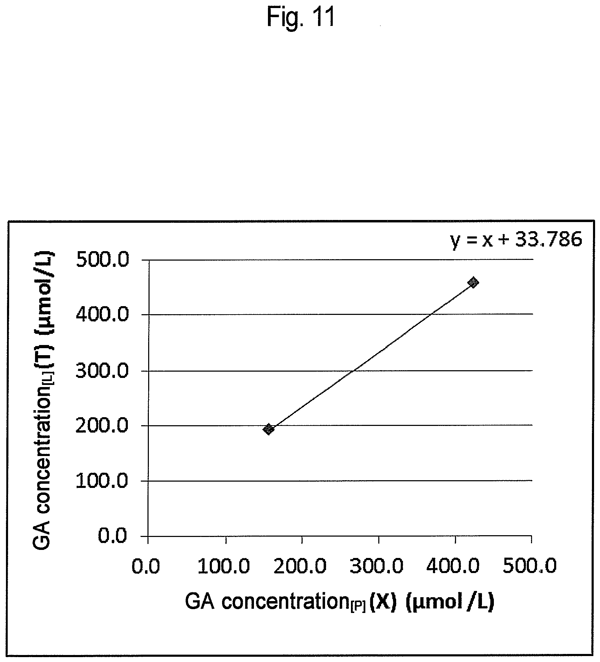

1) Regression equation (VI): y=x + 33.786 was set by plotting a glycated albumin concentration$_{[L]}$ (T) of the sample material as the vertical axis and a glycated albumin concentration$_{[P]}$ (X) as the horizontal axis (FIG. 11).

<Step of establishing a traceability system by converting, according to Linear regression equation (VI), a measured value of a sample material calculated using the reference material whose values were set according to Linear equation (II) >

1. A glycated albumin concentration$_{[L]}$ (R') was obtained by substituting, in Linear regression equation (VI), the glycated albumin concentration$_{[P]}$ (N') of the sample material whose values were measured in Example 10.

2. A glycated albumin value ($Q_3$') was calculated by dividing the glycated albumin concentration$_{[L]}$ (R') of the sample material by the albumin concentration (O') obtained in Example 10 and then multiplying the quotient by 1000.

Values obtained by converting, according to Linear regression equation (VI), the measured values of the sample material according to Linear equation (II) are listed in Table 12.

[Table 12]

| Item | Sample material |
| --- | --- |
| Glycated albumin value, set value (mmol/mol) | W: L 262, H 611 |
| Glycated albumin concentration$_{[L]}$ ($\mu$mol/L) | R': L 189.9, H 456.1 |
| Glycated albumin value (mmol/mol) | $Q_3$': L 262, H 611 |

As is apparent from Table 12, the glycated albumin value ($Q_3$') obtained by dividing, by the albumin concentration (O'), the glycated albumin concentration$_{[L]}$ (R') obtained by using the reference material whose values were set according to Linear equation (II) and converting the glycated albumin concentration$_{[P]}$ (N') of the glycated albumin certified reference material according to Linear regression equation (VI) agreed well with the glycated albumin value (W) set for the sample material in Example 10. This means that a series of sequential traceability system was established, judging from that a set value of the sample material set from the glycated albumin certified reference material is calculated by using the reference material whose values were set with the glycated albumin certified reference material as an upper level standard.

[Example 12]

In Example 12, in addition to the high reference material (H), Off the Clot Human Serum (Golden west Biologicals, Inc.) was used as a low reference material (L).

<Calculation of albumin concentration of reference material>

1) An albumin concentration (V-2) of the reference material was calculated by measuring an albumin-derived absorbance (U-2) of the reference material and substituting it in the linear regression equation (Equation 3).

<Setting of linear regression equation>

1) A glycated albumin concentration$_{[L]}$ (T-2) of the reference material was calculated by measuring a glycated albumin-derived absorbance (S-2) of the reference material and substituting it in Linear regression equation (I).

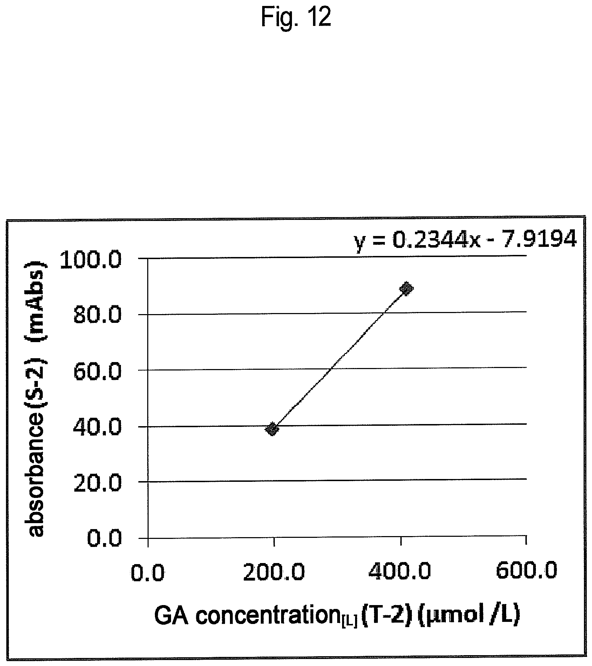

2) Linear regression equation (I-2): y=0.2344x - 7.9194 was set by plotting the glycated albumin concentration$_{[L]}$ (T-2) of the reference material as the horizontal axis and the glycated albumin-derived absorbance (S-2) as the vertical axis (FIG. 12).

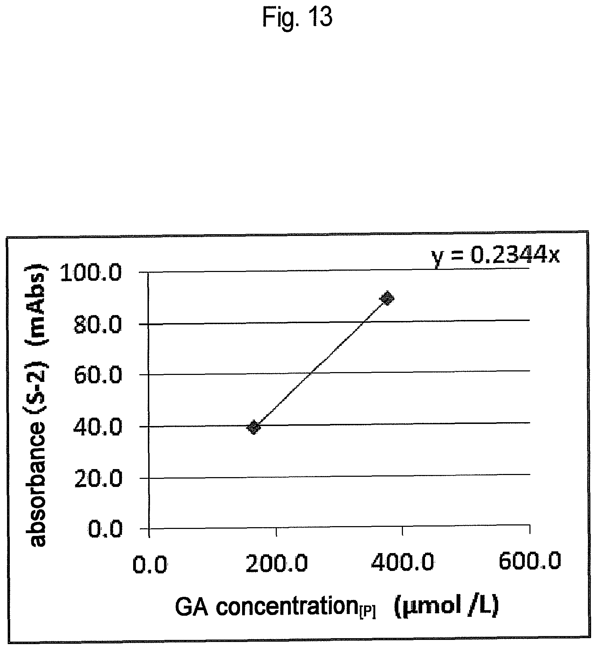

3) Linear equation (II-2): y=0.2344x was set by moving Linear regression equation (I-2) in parallel to give an intercept of 0 (FIG. 13).

<Setting of linear regression equation for conversion of measured value to certified value>
1) A glycated albumin concentration$_{[P]}$ (X-2) was calculated by substituting the glycated albumin-derived absorbance (S-2) in Linear equation (II-2) calculated above.
2) A glycated albumin value (W-2) (set value) was calculated by dividing the glycated albumin concentration$_{[L]}$ (T-2) by the albumin concentration (V-2) and then multiplying the quotient by 1000.
3) A glycated albumin value (Y-2) (measured value) was calculated by dividing the glycated albumin concentration$_{[P]}$ (X-2) by the albumin concentration (V-2) and then multiplying the quotient by 1000.
Values of low (L) and high (H) reference materials are listed in Table 13.

[Table 13]

| Item | Reference material |
|---|---|
| Albumin-derived absorbance (mAbs) | U-2: L 257.6, H 257.8 |
| Albumin concentration ($\mu$mol/L) | V-2: L 768.7, H 769.3 |
| Glycated albumin-derived absorbance (mAbs) | S-2: L 38.8, H 88.6 |
| Glycated albumin concentration$_{[L]}$ ($\mu$mol/L) | T-2: L 199.3, H 411.8 |
| Glycated albumin concentration$_{[P]}$ ($\mu$mol/L) | X-2: L 165.5, H 378.0 |
| Glycated albumin value (set value) (mmol/mol) | W-2: L 259.3, H 535.2 |
| Glycated albumin value (measured value) (mmol/mol) | Y-2: L 215.3, H 491.3 |

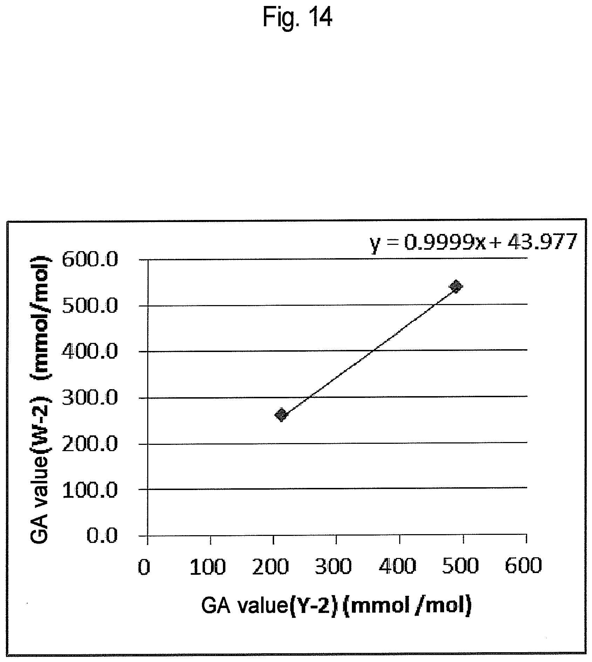

4) Linear regression equation (III-2): y=0.9999x + 43.977 was set by plotting the glycated albumin value (W-2) (set value) as the vertical axis and the glycated albumin value (Y-2) (measured value) as the horizontal axis. (FIG. 14).

<Step of obtaining a traceable value by substituting, in Linear regression equation (III-2), a measured value calculated using the reference material whose values were set according to Linear equation (II)>

1) A glycated albumin concentration$_{[P]}$ (N-2) of the glycated albumin certified reference material was measured by preparing a calibration curve with a reference material whose values were set according to Linear equation (II-2).

2) An albumin concentration (O-2) of the glycated albumin certified reference material was measured by preparing a calibration curve with the reference material whose values were set according to a linear regression equation (Equation 3).

3) A glycated albumin value (P-2) (measured value) was calculated by dividing the glycated albumin concentration$_{[P]}$ (N-2) of the glycated albumin certified reference material by the albumin concentration (O-2) and multiplying the quotient by 1000.

4) A glycated albumin value (Q-2) (converted value) was obtained by substituting the glycated albumin value (P-2) (measured value) in Linear regression equation (III-2).

Measured values of the glycated albumin certified reference material according to Linear equation (II-2) and converted values according to Linear regression equation (III-2) are listed in Table 14.

[Table 14]

| Item | Glycated albumin certified reference material |
|---|---|
| Certified glycated-albumin value (glycated albumin value) (mmol/mol) | B: M 230, H 365, HH 564 |
| Glycated albumin concentration$_{[P]}$ ($\mu$mol/L) | N-2: M 115.6, H 193.7, HH 296.1 |
| Albumin concentration ($\mu$mol/L) | O-2: M 650.3, H 622.2, HH 585.0 |
| Glycated albumin value, measured value (mmol/mol) | P-2: M 178, H 311, HH 506 |
| Glycated albumin value, converted value (mmol/mol) | Q-2: M 222, H 355, HH 550 |

As is apparent from Table 14, the converted value (Q-2) obtained by converting, according to Linear regression equation (III-2), the measured value (P-2) of the glycated albumin certified reference material calculated using the reference material whose values were set according to Linear equation (II-2) agreed well with the certified value (B) specified for the glycated albumin certified reference material. This means that a series of sequential traceability system can be established, judging from that the certified value of the glycated albumin certified reference material can be obtained by using the reference material whose values were set with the glycated albumin certified reference material as an upper level standard.

[Example 13]

A test was made using, in addition to the glycated albumin certified reference material and the albumin certified reference material used in Example 1, the followings.

1) Reagent for measurement

As a reagent for measuring the absorbance of albumin (ALB) and glycated albumin, a glycated albumin measuring reagent (Beijing Strong Biotechnologies, Inc.) was used.

The glycated albumin reagent of this company is composed of a first reagent and a second reagent and the ALB reagent (BCG method) is composed of a first reagent.

2) Reference material

As a reference material, a calibrator (Beijing Strong Biotechnologies, Inc.) was used.

3) Measurement apparatus

As an apparatus for measurement of absorbance and conversion from absorbance to concentration, a Hitachi 7180 Automatic Analyzer (Hitachi High-Tech Fielding) was used.

4) Glycated albumin measurement method

By using the measurement apparatus described above in 3), measurement was performed in a 10-min mode with 3.0 μL of a sample, 160 μL of a first reagent, and 40 μL of a second reagent. At photometry point 0, sampling and stirring of the sample and the first reagent were performed, measurement was started, and a first reaction was carried out. Then, after sampling and stirring of the second reagent at point 16, a second reaction was carried out. Measurement was performed until 34 points in total.

The other operation was performed based on the package insert or instruction manual.

5) Calculation method of glycated albumin absorbance

The glycated albumin absorbance (change in measured absorbance of measurement sample) was determined according to the following calculation equation, while using an average absorbance between points 33 and 34 and an average absorbance between points 15 and 16 before addition of the second reagent.

$$\Delta mAbs = (Ab1-(Ab2\times0.8))/10$$

ΔmAbs: change in absorbance of glycated albumin

Ab1: average absorbance between points 33 and 34

Ab2: average absorbance between points 15 and 16

In the above equation, "0.8" is a value calculated according to the following calculation equation: amount of first reagent (160 μL)/(amount of first reagent (160 μL) + amount of second reagent (40 μL)) = 0.8

6) Albumin measurement method

By using the measurement apparatus described above in 3), measurement was performed in a 5-min mode with 2.0 μL of a sample and 200 μL of the first reagent. At point 0, sampling and stirring of the sample and the first reagent were performed, measurement was started, and a reaction was carried out. Measurement was performed until 17 points in total.

The other operation was performed based on the package insert or instruction manual.

7) Calculation method of albumin (ALB) absorbance

An ALB absorbance (change in measured absorbance of measurement sample) was determined according to the following calculation equation, while using an average absorbance between points 8 and 9.

$$\Delta mAbs = (Ab1)/10$$

$\Delta mAbs$: change in absorbance of ALB

Ab1: average absorbance between points 8 and 9

<Setting of Linear regression equation ($I_{(a)}$)>

1) Values as a result of investigation in a manner similar to Example 1 are listed in Table 15.

[Table 15]

| Item | Glycated albumin certified reference material | Albumin certified reference material |
|---|---|---|
| Certified glycated-albumin value (glycated albumin value) (mmol/mol) | B: M 230, H 365, HH 564 | - |
| Certified albumin value (albumin concentration)(g/dL) | - | 3.72 g/dL |
| Molar certified albumin value (albumin concentration) ($\mu$mol/L) | - | $L_{(a)}$: 559.9 $\mu$mol/L |
| Albumin absorbance (mAbs) | $K_{(a)}$: M 1019.0, H 1003.7, HH 936.2 | $M_{(a)}$: 867.4 |
| Albumin concentration ($\mu$mol/L) | $C_{(a)}$: M 657.8, H 647.9, HH 604.4 | - |
| Glycated albumin-derived absorbance (mAbs) | $D_{(a)}$: M 18.6, H 30.2, HH 44.6 | - |
| Glycated albumin concentration$_{[L]}$ ($\mu$mol/L) | $A_{(a)}$: M 151.3, H 236.5, HH 340.9 | - |

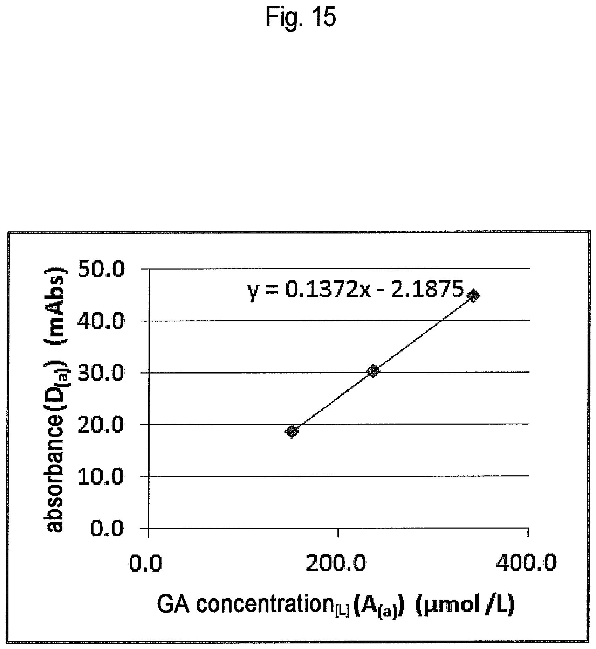

2) Linear regression equation ($I_{(a)}$): y=0.1372x - 2.1875 was set by plotting the glycated albumin concentration$_{[L]}$ ($A_{(a)}$) of the glycated albumin certified reference material as the horizontal axis and the glycated albumin absorbance ($D_{(a)}$) as the vertical axis (FIG. 15).
<Setting of a linear regression equation obtained by making the intercept of Linear regression equation ($I_{(a)}$) zero>

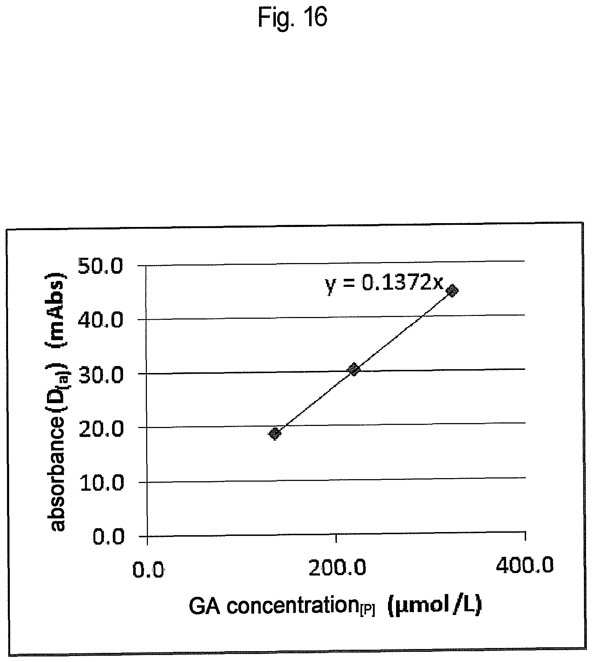

1) Linear equation ($II_{(a)}$): y = 0.1372 $\times$ ($II_{(a)}$) was set by moving Linear regression equation ($I_{(a)}$) in parallel to give an intercept of 0 (FIG.16).

<Setting of Linear regression equation ($III_{(a)}$)>

1) Values as a result of investigation in a manner similar to Example 2 are listed in Table 16.

[Table 16]

| Item | Glycated albumin certified reference material |
|---|---|
| Certified glycated-albumin value (glycated albumin value) (mmol/mol) | B: M 230, H 365, HH 564 |
| Glycated albumin concentration$_{[P]}$ ($\mu$mol/L) | $E_{(a)}$: M 135.6, H 220.1, HH 325.1 |
| Albumin concentration ($\mu$mol/L) | $C_{(a)}$: M 657.8, H 647.9, HH 604.4 |
| Glycated albumin value, measured value (mmol/mol) | $F_{(a)}$: M 206, H 340, HH 538 |

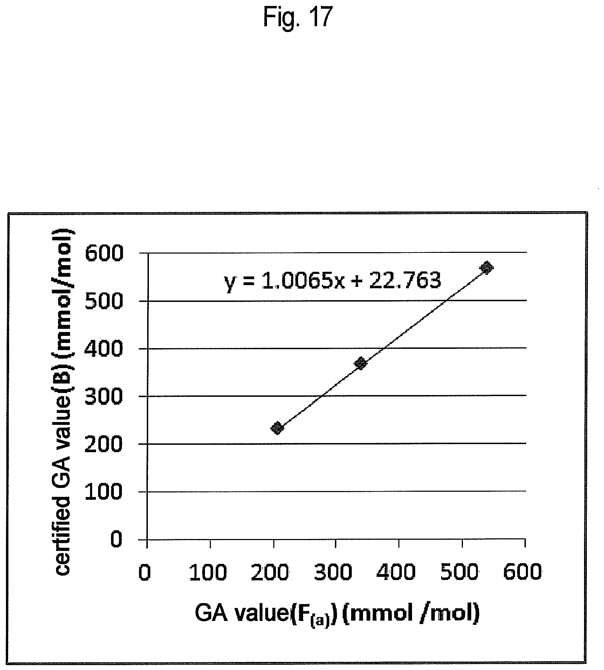

2) Linear regression equation $(III_{(a)})$: y=1.0065x + 22.763 was set by plotting the certified glycated-albumin value (B) of the glycated albumin certified reference material as the vertical axis and the glycated albumin value $(F_{(a)})$ as the horizontal axis (FIG. 17).

<Setting of values for the reference material according to Linear regression equation $(III_{(a)})$>

1) Values as a result of investigation in a manner similar to Example 3 are listed in Table 17.

[Table 17]

| Item | Reference material |
|---|---|
| Albumin-derived absorbance (mAbs) | $I_{(a)}$: 986.7 |
| Albumin concentration ($\mu$mol/L) | $J_{(a)}$: 637.0 |
| Glycated albumin absorbance (mAbs) | $G_{(a)}$: 45.9 |
| Glycated albumin concentration$_{[P]}$ ($\mu$mol/L) | $H_{(a)}$: 334.5 |

<Establishment of traceability according to Linear regression equation $(III_{(a)})$> Values as a result of investigation in a manner similar to Example 4 are listed in Table 18.

[Table 18]

| Item | Glycated albumin certified reference material |
|---|---|
| Certified glycated-albumin value (glycated albumin value) (mmol/mol) | B: M 230, H 365, HH 564 |
| Glycated albumin concentration$_{[P]}$ ($\mu$mol/L) | $N_{(a)}$: M 135.6, H 220.1, HH 325.1 |
| Albumin concentration ($\mu$mol/L) | $O_{(a)}$: M 657.8, H 647.9, HH 604.4 |
| Glycated albumin value, measured value (mmol/mol) | $P_{(a)}$: M 206, H 340, HH 538 |
| Glycated albumin value, converted value (mmol/mol) | $Q_{(a)}$: M 230, H 365, HH 564 |

As is apparent from Table 18, the converted value ($Q_{(a)}$) obtained, in a manner similar to that of Example 4, by converting, according to Linear regression equation $(III_{(a)})$, the measured value ($P_{(a)}$) of the glycated albumin certified reference material calculated from the reference material whose values were set according to Linear equation $(II_{(a)})$ agreed well with the certified value (B) specified for the glycated albumin certified reference material. This means that a series of sequential traceability system can be established, judging from that the certified value of the glycated albumin certified reference material can be obtained by using a reference material whose values were set with the glycated albumin certified reference material as an upper level standard.

[Example 14]

A test was made using, in addition to the glycated albumin certified reference material and the albumin certified reference material used in Example 1, the followings.

1) Measurement reagent

As a reagent for measuring the absorbance of albumin (ALB) and glycated albumin, Glycated serum albumin (IVDLab Co., LTD) was used.

Glycated serum albumin for a glycated albumin reagent is composed of a first reagent and a second reagent and that for an ALB reagent (BCG method) is composed of a first reagent.

2) Reference material

As a reference material, Glycated albumin Calibrator (IVDLab CO., LTD) was used.

3) Measurement apparatus

As an apparatus for measurement of an absorbance and conversion from absorbance to concentration, a Hitachi 7180 Automatic Analyzer (Hitachi High-Tech Fielding) was used.

4) Glycated albumin measurement method

By using the measurement apparatus described above in 3), measurement was performed in a 10-min mode with 10.0 μL of a sample, 200 μL of a first reagent, and 50 μL of a second reagent. At photometry point 0, sampling and stirring of the sample and the first reagent were performed, measurement was started, and a first reaction was carried out. Then, after sampling and stirring of the second reagent at point 16, a second reaction was carried out. Measurement was performed until 34 points in total.

The other operation was performed based on the package insert or instruction manual.

5) Calculation method of glycated albumin absorbance

The glycated albumin absorbance (change in measured absorbance of measurement sample) was determined according to the following calculation equation, while using an average absorbance between points 33 and 34 and an average absorbance between points 15 and 16 before addition of the second reagent.

$$\Delta mAbs = (Ab1-(Ab2\times0.8))/10$$

ΔmAbs: change in absorbance of glycated albumin

Ab1: average absorbance between points 33 and 34

Ab2: average absorbance between points 15 and 16

In the above equation, "0.8" is a value calculated according to the following calculation equation: amount of first reagent (200 μL)/(amount of first reagent (200 μL) + amount of second reagent (50 μL)) = 0.8

6) Albumin measurement method

By using the measurement apparatus described above in 3), measurement was performed in a 1-min mode with 2.0 μL of the sample and 200 μL of the first reagent. At point 0, sampling and stirring of the sample and the first reagent were performed, measurement was started, and a reaction was carried out. Measurement was performed until 4 points in total.

The other operation was performed based on the package insert or instruction manual.

7) Calculation method of albumin (ALB) absorbance

An ALB absorbance (change in measured absorbance of measurement sample) was determined according to the following calculation equation, while using an average absorbance between points 3 and 4.

$$\Delta mAbs = (Ab1)/10$$

ΔmAbs: change in absorbance of ALB

Ab1: average absorbance between points 3 and 4

<Setting of Linear regression equation ($I_{(b)}$)>

1) Values as a result of investigation in a manner similar to Example 1 are listed in Table 19.

[Table 19]

| Item | Glycated albumin certified reference material | Albumin certified reference material |
|---|---|---|
| Certified glycated-albumin value (glycated albumin value) (mmol/mol) | B: M 230, H 365, HH 564 | - |
| Certified albumin value (albumin concentration) (g/dL) | - | 3.72 g/dL |
| Molar certified albumin value (Albumin concentration) ($\mu$mol/L) | - | $L_{(b)}$: 559.9 $\mu$mol/L |
| Albumin absorbance (mAbs) | $K_{(b)}$: M 996.5, H 974.5, HH 907.3 | $M_{(b)}$: 845.9 |
| Albumin concentration ($\mu$mol/L) | $C_{(b)}$: M 659.6, H 645.0, HH 600.5 | - |
| Glycated albumin absorbance (mAbs) | $D_{(b)}$: M 37.4, H 61.4, HH 91.4 | - |
| Glycated albumin concentration$_{[L]}$ ($\mu$mol/L) | $A_{(b)}$: M 151.7, H 235.4, HH 338.7 | - |

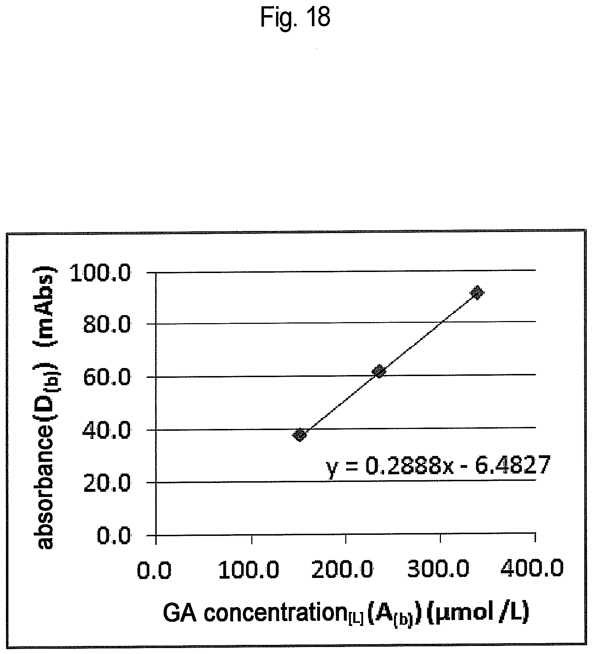

2. Linear regression equation ($I_{(b)}$): y=0.2888x - 6.4827 was set by plotting the glycated albumin concentration$_{[L]}$ ($A_{(b)}$) of the glycated albumin certified reference material as the horizontal axis and the glycated albumin absorbance ($D_{(b)}$) as the vertical axis (FIG. 18).

<Setting of linear regression equation by making the intercept of Linear regression equation ($I_{(b)}$) zero>

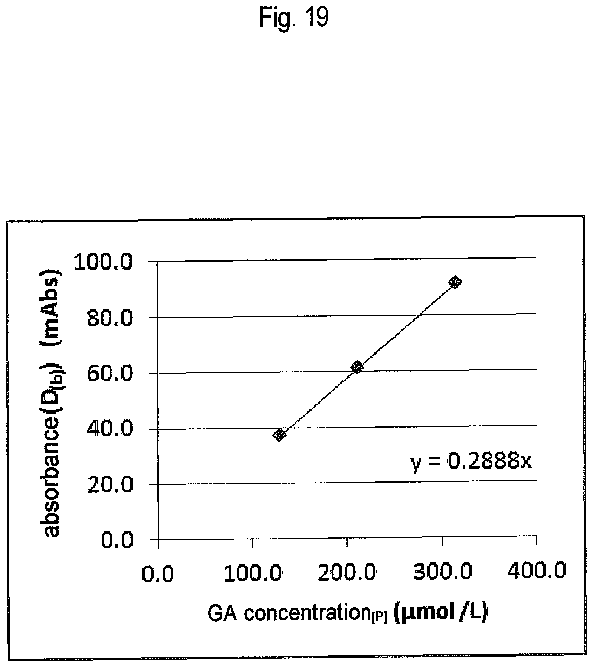

1) Linear equation ($II_{(b)}$): y=0.2888x ($II_{(b)}$) was set by moving Linear regression equation ($I_{(b)}$) in parallel to give an intercept of 0 (FIG. 19).

<Setting of Linear regression equation ($III_{(b)}$)>

1) Values as a result of investigation in a manner similar to Example 2 are listed in Table 20.

[Table 20]

| Item | Glycated albumin certified reference material |
|---|---|
| Certified glycated-albumin value (glycated albumin value) (mmol/mol) | B: M 230, H 365, HH 564 |
| Glycated albumin concentration$_{[P]}$ ($\mu$mol/L) | $E_{(b)}$: M 129.5, H 212.6, HH 316.5 |
| Albumin concentration ($\mu$mol/L) | $C_{(b)}$: M 659.6, H 645.0, HH 600.5 |
| Glycated albumin value, measured value (mmol/mol) | $F_{(b)}$: M 196, H 330, HH 527 |

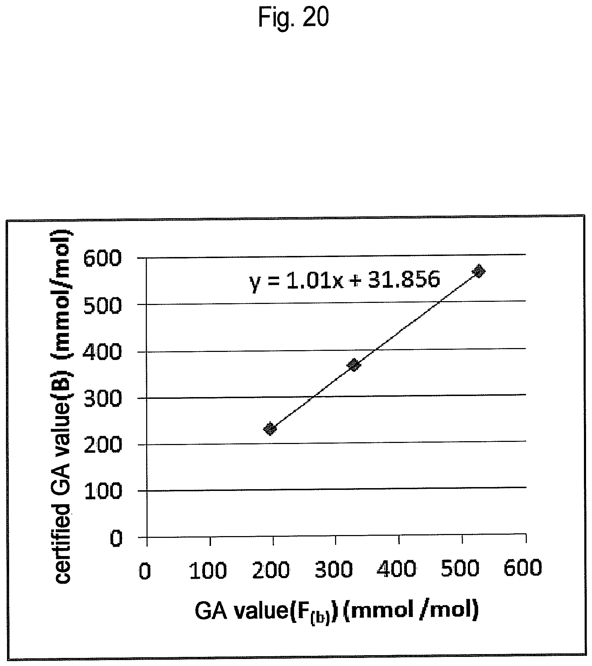

2) Linear regression equation ($III_{(b)}$): y=1.01x + 31.856 was set by plotting the certified glycated-albumin value of the glycated albumin certified reference material as the vertical axis and the glycated albumin value ($F_{(b)}$) as the horizontal axis (FIG. 20).

<Setting of values for reference material according to Linear regression equation ($III_{(b)}$)>

Values as a result of investigation in a manner similar to Example 3 are listed in Table 21.

[Table 21]

| Item | Reference material |
|---|---|
| Albumin-derived absorbance (mAbs) | $I_{(b)}$: 936.3 |
| Albumin concentration ($\mu$mol/L) | $J_{(b)}$: 619.7 |
| Glycated albumin absorbance (mAbs) | $G_{(b)}$: 80.4 |
| Glycated albumin concentration$_{[P]}$ ($\mu$mol/L) | $H_{(b)}$: 278.4 |

<Establishment of traceability according to Linear regression equation (III$_{(b)}$)>

Values as a result of investigation in a manner similar to Example 4 are listed in Table 22.

[Table 22]

| Item | Glycated albumin certified reference material |
| --- | --- |
| Certified glycated-albumin value (glycated albumin value) (mmol/mol) | B: M 230, H 365, HH 564 |
| Glycated albumin concentration$_{[P]}$ ($\mu$mol/L) | N$_{(b)}$: M 129.5, H 212.6, HH 316.5 |
| Albumin concentration ($\mu$mol/L) | O$_{(b)}$: M 659.6, H 645.0, HH 600.5 |
| Glycated albumin value, measured value (mmol/mol) | P$_{(b)}$: M 196, H 330, HH 527 |
| Glycated albumin value, converted value (mmol/mol) | Q$_{(b)}$: M 230, H 365, HH 564 |

As is apparent from Table 22, the converted value (Q$_{(b)}$) obtained, in a manner similar to Example 4, by converting, according to Linear regression equation (III$_{(b)}$), the measured value (P$_{(b)}$) of the glycated albumin certified reference material calculated from the reference material whose values were set according to Linear equation (II$_{(b)}$) agreed well with the certified value (B) specified for the glycated albumin certified reference material. This means that a series of sequential traceability system can be established, judging from that the certified value of the glycated albumin certified reference material can be obtained by using a reference material whose values were set with the glycated albumin certified reference material as an upper level standard.

[Comparative Example 1]

<Setting of a value for a reference material according to Linear regression equation (I)>

1) A glycated albumin-derived absorbance (G) of a reference material was measured.

2) A glycated albumin concentration$_{[L]}$ (T$_{(C)}$) of the reference material was calculated by substituting the glycated albumin absorbance (G) in Linear regression equation (I).

3) An albumin-derived absorbance (I) of the reference material was measured.

4) An albumin concentration (J) of the reference material was calculated by substituting the albumin absorbance (I) in the linear regression equation (Equation 3) of Example 3.

Values of the reference material determined above are listed in Table 23.

[Table 23]

| Item | Reference material |
| --- | --- |
| Albumin absorbance (mAbs) | I: 257.8 |
| Albumin concentration ($\mu$mol/L) | J: 769.3 |
| Glycated albumin absorbance (mAbs) | G: 88.6 |
| Glycated albumin concentration$_{[L]}$ ($\mu$mol/L) | T$_{(c)}$: 378.0 |

<Measurement results of glycated albumin certified reference material using reference material whose values were set according to Linear regression equation (I)>

1) A glycated albumin concentration$_{[L]}$ (N$_{(C)}$) of a glycated albumin certified reference material was measured by using a reference material whose values were set according to Linear regression equation (I) and preparing a calibration curve for converting a glycated albumin absorbance into a glycated albumin concentration.

2) An albumin concentration (O) of the glycated albumin certified reference material was measured by using the reference material whose values were set according to Linear regression equation (I) and preparing a calibration curve for converting an albumin absorbance into an albumin concentration.

3) A glycated albumin value (P$_{(C)}$) was calculated by dividing the glycated albumin concentration$_{[L]}$ (N$_{(C)}$) of the glycated albumin certified reference material by the albumin concentration (O) and then multiplying the quotient by 1000. Values of the glycated albumin certified reference material according to Linear regression equation (I) are listed in Table 24.

[Table 24]

| Item | Glycated albumin certified reference material |
|---|---|
| Certified glycated-albumin value (glycated albumin value) (mmol/mol) | B: M 230, H 365, HH 564 |
| Glycated albumin concentration$_{[L]}$ ($\mu$mol/L | N$_{(c)}$: M 125.9, H 211.0, HH 322.5 |
| Albumin concentration ($\mu$mol/L) | O: M 650.3, H 622.2, HH 585.0 |
| Glycated albumin value, measured value (mmol/mol) | P$_{(c)}$: M 194, H 339, HH 551 |

4) As is apparent from Table 24, the measured value (P$_{(C)}$) of the glycated albumin value of the glycated albumin certified reference material did not agree with the certified value (B) specified for the glycated albumin certified reference material even if the reference material whose values were set according to Linear regression equation (I) was used. This means that a series of sequential traceability system cannot be established, judging from that the certified value of the glycated albumin certified reference material cannot be obtained by calculation using a reference material whose values are set by the method described in Examples, with the glycated albumin certified reference material as an upper level standard.

Industrial Applicability

The present invention can provide a method capable of making measured values, when they do not agree with each other due to a difference in measurement method, agree with each other. For example, the present invention makes it possible to measure a GA value of a sample material traceable to a certified GA value by the enzymatic method combined with absorbance measurement permitting simple, inexpensive and short-time measurement. The invention is particularly useful for the diagnosis and management of diseases or states associated with a GA value, such as diabetes and therefore has industrial applicability in the medical field, reagent field and the like.
This application claims the priority of Japanese Patent Application No. 2016-148973, filed on July 28, 2016 which is hereby incorporated by reference.

**Claims**

**1.**　A method of measuring a glycated-albumin (GA) value traceable to a certified GA value, the method comprising the following steps:

A$_0$) a step of determining a regression equation for converting:

a measured value of a GA value of a sample material into a certified GA value of a GA certified reference material;
a measured value of a GA concentration$_{[P]}$ of the sample material into a GA concentration$_{[L]}$ of the GA certified reference material;
the measured value of the GA value of the sample material into a GA value set for the sample material from the certified GA value of the GA certified reference material; or
the measured value of the GA concentration$_{[P]}$ of the sample material into a GA concentration$_{[L]}$ set for the sample material from the GA concentration$_{[L]}$ of the GA certified reference material; and

F$_0$) a step of measuring a GA concentration$_{[P]}$ and an albumin concentration of the sample material by using a calibration curve prepared by using a reference material for which "a GA concentration$_{[P]}$ converted from the GA concentration$_{[L]}$ of the GA certified reference material" and "an albumin concentration converted from an albumin concentration of the GA certified reference material" are set and determining a GA value traceable to the certified GA value according to the regression equation determined in Step A$_0$)
(in each step, the GA concentration$_{[L]}$ is a concentration of glycated lysine residues (DOF-Lys) and the GA concentration$_{[P]}$ is a concentration of a glycated amino acid or glycated peptide).

2. A method of measuring a glycated-albumin (GA) value traceable to a certified GA value, the method comprising the following steps:

$A_1$) a step of determining Regression equation III by steps including the following b), c), and $e_0$) to h):

b) a step of determining respective GA concentrations$_{[L]}$ (A) of two or more GA certified reference materials having respectively different certified GA values according to the following Equation 1:

$$GA\ concentration_{[L]}\ (A) = (certified\ GA\ value\ (B)\ of\ GA\ certified\ reference$$

$$material) \times (albumin\ concentration\ (C)\ of\ GA\ certified\ reference\ material)$$

$$(Equation\ 1)$$

c) a step of measuring respective GA-derived absorbances (D) of the two or more GA certified reference materials;

$e_0$) a step of creating the following linear equation $II_0$ having an intercept of zero:

$$D = \alpha_0 E \qquad (Equation\ II_0);$$

(wherein,

$\alpha_0$ is an arbitrary positive value and
E represents a GA concentration$_{[P]}$ (E) proportionate to the GA-derived absorbance (D)),

f) a step of determining two or more GA concentrations$_{[P]}$ (E) proportionate to the GA-derived absorbances (D) according to the Equation $II_0$;

g) a step of determining two or more GA values (F) according to the following Equation 2:

$$GA\ value\ (F) = (GA\ concentration_{[P]}\ (E))/(albumin\ concentration\ (C))$$

$$(Equation\ 2);$$

and

h) a step of creating the following Regression equation III from the respective certified GA values (B) of the two or more GA certified reference materials and the two or more GA values (F) corresponding thereto:

$$B = \gamma F + \delta \qquad (Regression\ equation\ III)$$

(wherein in each of the steps, the GA concentration$_{[L]}$ is a concentration of glycated lysine residues (DOF-Lys) and the GA concentration$_{[P]}$ is a concentration of a glycated amino acid or a glycated peptide).

3. The method according to Claim 2, further comprising the following steps:

B) a step of measuring a GA-derived absorbance (G) of a reference material and determining a GA concentration$_{[P]}$ (H) corresponding thereto according to the Equation $II_0$, and
C) a step of measuring an albumin-derived absorbance (I) of the reference material and determining an albumin concentration (J) of the reference material.

4. The method according to Claim 3, wherein Step C) includes the following Steps C1) to C3):

C1) a step of determining $\varepsilon$ that satisfies the following Equation 3 from an albumin-derived absorbance (K) of the GA certified reference material and the albumin concentration (C) of the GA certified reference material:

$$K=\varepsilon C \quad \text{(Equation 3)};$$

C2) a step of measuring the albumin-derived absorbance (I) of the reference material; and
C3) a step of determining the albumin concentration (J) of the reference material from the absorbance (I) according to the following Equation 3':

$$I=\varepsilon J \quad \text{(Equation 3')}.$$

5. The method according to Claim 3, further comprising, prior to Step b) the following steps a1) to a3):

   a1) a step of determining a molar certified albumin value (L) according to the following Equation 4:

   molar certified albumin value (L) = (certified albumin value of albumin certified

   reference material)/(molecular weight of albumin)    (Equation 4);

   a2) a step of measuring an albumin-derived absorbance (M) of the albumin certified reference material; and
   a3) a step of determining $\theta$ that satisfies the following Equation 5 from the molar certified albumin value (L) and the absorbance (M):

   $$M=\theta L \quad \text{(Equation 5)};$$

   and
   Step (C) includes the following Steps C2) and C4):

   C2) a step of measuring the albumin-derived absorbance (I) of the reference material; and
   C4) a step of determining the albumin concentration (J) of the reference material from the absorbance (I) according to the following Equation 5':

   $$I=\theta J \quad \text{(Equation 5')}.$$

6. The method according to any one of Claims 2 to 5, further comprising the following Steps F1) to F4):

   F1) a step of determining, from the GA-derived absorbance (D) of the GA certified reference material, a GA concentration$_{[P]}$ (N) corresponding thereto by using a calibration curve prepared by using the reference material;
   F2) a step of determining, from the albumin-derived absorbance (K) of the GA certified reference material used in Step F1), an albumin concentration (O) corresponding thereto by using a calibration curve prepared by using the reference material;
   F3) a step of determining a GA value (P) according to the following Equation 6:

   GA value (P) of GA certified reference material = (GA concentration$_{[P]}$

   (N))/(albumin concentration (O))    (Equation 6)

   and
   F4) a step of determining a GA value (Q) traceable to the certified value of the GA certified reference material used in Step F1) while using the GA value (P) and the Regression equation III.

7. A method of measuring a glycated-albumin (GA) value traceable to a certified GA value, the method comprising the following steps:
   A$_2$) a step of determining Regression equation IV by carrying out steps including the following b), c), e$_0$), f), and i):

b) a step of determining respective GA concentrations$_{[L]}$ (A) of two or more GA certified reference materials having respectively different certified GA values according to the following Equation 1:

$$\text{GA concentration}_{[L]} \text{ (A)} = \text{(certified GA value (B) of GA certified reference material)} \times \text{(albumin concentration (C) of GA certified reference material)}$$

(Equation 1);

c) a step of measuring respective GA-derived absorbances (D) of two or more GA certified reference materials;
e$_0$) a step of creating the following Linear Equation II$_0$ having an intercept of zero:

$$D=\alpha_0 E \quad \text{(Equation II}_0),$$

(wherein,

> $\alpha_0$ is an arbitrary positive value and
> E is a GA concentration$_{[P]}$ (E) proportionate to the GA-derived absorbance (D));

f) a step of determining two or more GA concentrations$_{[P]}$ (E) proportionate to the GA-derived absorbances (D) according to the Equation II$_0$; and
i) a step of creating, from the two or more GA concentrations$_{[L]}$ (A) and GA concentrations$_{[P]}$ (E), the following Regression equation IV:

$$A=\kappa E+\lambda \quad \text{(Regression equation IV)}$$

(wherein in each of the above-described steps, the GA concentration$_{[L]}$ is a concentration of glycated lysine residues (DOF-Lys) and the GA concentration$_{[P]}$ is a concentration of a glycated amino acid or a glycated peptide).

8. The method according to Claim 7, further comprising the following steps:

> B) a step of measuring a GA-derived absorbance (G) of a reference material and determining a GA concentration$_{[P]}$ (H) corresponding thereto according to the Equation II$_0$; and
> C) a step of measuring an albumin-derived absorbance (I) of the reference material and determining an albumin concentration (J) of the reference material.

9. The method according to Claim 7 or 8, further comprising the following steps:

> F1) a step of determining, from the GA-derived absorbance (D) of the GA certified reference material, a GA concentration$_{[P]}$ (N) in the enzymatic method corresponding thereto by using a calibration curve prepared by using the reference material;
> F2) a step of determining, from an albumin-derived absorbance (K) of the GA certified reference material used in Step F1), an albumin concentration (O) corresponding thereto by using a calibration curve prepared by using the reference material;
> F5) a step of determining, from the GA concentration$_{[P]}$ (N), a GA concentration$_{[L]}$ (R) of the GA certified reference material according to the Regression equation IV; and
> F6) a step of determining a GA value (Q$_1$') traceable to the certified value of the GA certified reference material used in Step F1) according to the following Equation 7:

$$\text{GA value } (Q_1') \text{ of GA certified reference material} = (\text{GA concentration}_{[L]}$$

$$(R))/(\text{albumin concentration } (O)) \quad \text{(Equation 7)}.$$

10. A method of measuring a glycated-albumin (GA) value traceable to the certified GA value, the method comprising the following steps:
    $A_3$) a step of determining Regression equation V by steps including the following b) to $e_0$) and j) to o):

    b) a step of determining respective GA concentrations$_{[L]}$ (A) of two or more GA certified reference materials having respectively different certified GA values according to the following Equation 1:

    $$\text{GA concentration}_{[L]} (A) = (\text{certified GA value } (B) \text{ of GA certified reference}$$

    $$\text{material}) \times (\text{albumin concentration } (C) \text{ of GA certified reference material})$$

    $$\text{(Equation 1)};$$

    c) a step of measuring respective GA-derived absorbances (D) of the two or more GA certified reference materials;
    d) a step of creating, from the two or more GA-derived absorbances (D) and the two or more GA concentrations$_{[L]}$ (A), the following Regression equation I:

    $$D = \alpha A + \beta \quad \text{(Regression equation I)};$$

    $e_0$) a step of creating the following Linear equation $II_0$ having an intercept of zero:

    $$D = \alpha_0 E \quad \text{(Equation II}_0)$$

    (wherein,

    $\alpha_0$ is an arbitrary positive value and
    E represents a GA concentration$_{[P]}$ (E) proportionate to the GA-derived absorbance (D)),

    j) a step of measuring respective GA-derived absorbances (S) of two or more sample materials and determining GA concentrations$_{[L]}$ (T) corresponding thereto according to the Regression equation I;
    k) a step of measuring respective albumin-derived absorbances (U) of the two or more sample materials and determining albumin concentrations (V):

    l) a step of determining, from the GA concentrations$_{[L]}$ (T), respective GA values$_1$ (W) of the two or more sample materials according to the following Equation 8:

    $$\text{GA value}_1 (W) = (\text{GA concentration}_{[L]} (T))/(\text{albumin concentration } (V))$$

    $$\text{(Equation 8)};$$

    m) a step of determining, from the respective GA-derived absorbances (S) of the two or more sample materials, GA concentrations$_{[P]}$ (X) corresponding thereto according to the Equation $II_0$;
    n) a step of determining, from the GA concentrations$_{[P]}$ (X), respective GA values$_2$ (Y) of the two or more sample materials according to the following Equation 9:

$$\text{GA value}_2 \text{ (Y)} = (\text{GA concentration}_{[P]} \text{ (X)})/(\text{albumin concentration (V)})$$

$$\text{(Equation 9);}$$

and

o) a step of creating, from the GA values$_1$ (W) of the two or more sample materials and the two or more GA values$_2$ (Y) corresponding thereto, the following Regression equation V:

$$W = \pi Y + \varphi \qquad \text{(Regression equation V)}$$

(wherein in each step, the GA concentration$_{[L]}$ is a concentration of glycated lysine residues (DOF-Lys) and the GA concentration$_{[P]}$ is a concentration of a glycated amino acid or a glycated peptide).

**11.** The method according to Claim 10, further comprising the following steps:

B) a step of measuring a GA-derived absorbance (G) of a reference material and determining a GA concentration$_{[P]}$ (H) corresponding thereto according to the Equation II$_0$; and

C) a step of measuring an albumin-derived absorbance (I) of the reference material and determining an albumin concentration (J) of the reference material.

**12.** The method according to Claim 10 or 11, further comprising the following steps:

F1') a step of determining, from the GA-derived absorbance (S) of the sample material, a GA concentration$_{[P]}$ (N') corresponding thereto by using a calibration curve prepared by using the reference material;

F2') a step of determining, from the albumin-derived absorbance (U) of the sample material used in Step F1'), an albumin concentration (O') corresponding thereto by using a calibration curve prepared by using the reference material;

F3') a step of determining a GA value (P') according to the following Equation 6':

$$\text{GA value (P') of sample material} = (\text{GA concentration}_{[P]} \text{ (N')})/(\text{albumin concentration (O')}) \qquad \text{(Equation 6');}$$

and

F4') a step of determining, by using the GA value (P') and the Regression equation V, a GA value (Q$_2$') traceable to a certified GA value$_1$ (W) with which the sample material used in Step F1') is assigned.

**13.** A method of measuring a glycated-albumin (GA) value traceable to a certified GA value, the method comprising the following steps:

A$_4$) a step of determining Regression equation VI by steps including the following b) to e$_0$), j), m), and p):

b) a step of determining respective GA concentrations$_{[L]}$ (A) of two or more GA certified reference materials having respectively different certified GA values according to the following Equation 1:

$$\text{GA concentration}_{[L]} \text{ (A)} = (\text{certified GA value (B) of GA certified reference material}) \times (\text{albumin concentration (C) of GA certified reference material}) \qquad \text{(Equation 1);}$$

c) a step of measuring respective GA-derived absorbances (D) of the two or more GA certified reference materials;

d) a step of creating, from the two or more GA-derived absorbances (D) and the two or more GA concentrations$_{[L]}$ (A), Regression equation I:

$$D=\alpha A+\beta \quad \text{(Regression equation I);}$$

eo) a step of creating the following linear Equation II$_0$ having an intercept of zero:

$$D=\alpha_0 E \quad \text{(Equation II}_0)$$

(wherein,

$\alpha_0$ is an arbitrary positive value and
E is a GA concentration$_{[P]}$ (E) proportionate to the GA-derived absorbance (D));

j) a step of measuring respective GA-derived absorbances (S) of two or more sample materials and determining GA concentrations$_{[L]}$ (T) corresponding thereto according to the Regression equation I;
m) a step of determining, from the respective GA-derived absorbances (S) of the two or more sample materials, GA concentrations$_{[P]}$ (Z) corresponding thereto according to the Equation II$_0$;
p) a step of creating, from the GA concentrations$_{[L]}$ (T) and GA concentrations$_{[P]}$ (X) of the two or more sample materials, Regression equation VI:

$$T=\psi X+\omega \quad \text{(Regression equation VI)}$$

(wherein in each step, the GA concentration$_{[L]}$ is a concentration of glycated lysine residues (DOF-Lys) and the GA concentration$_{[P]}$ is a concentration of a glycated amino acid or a glycated peptide).

**14.** The method according to Claim 13, further comprising the following steps:

B) a step of measuring a GA-derived absorbance (G) of a reference material and determining a GA concentration$_{[P]}$ (H) corresponding thereto according to the Equation II$_0$; and
C) a step of measuring an albumin-derived absorbance (I) of the reference material and determining an albumin concentration (J) of the reference material.

**15.** The method according to Claim 13 or 14,
wherein the method further comprises, prior to Step b), the following steps a1) to a3):

a1) a step of determining a molar certified albumin value (L) according to the following Equation 4:

$$\text{molar certified albumin value (L)} = \text{(certified albumin value of albumin certified}$$

$$\text{reference material)/(molecular weight of albumin)} \quad \text{(Equation 4);}$$

a2) a step of measuring an albumin-derived absorbance (M) of the albumin certified reference material; and
a3) a step of determining, from the molar certified albumin value (L) and the absorbance (M), $\theta$ that satisfies the following Equation 5:

$$M=\theta L \quad \text{(Equation 5);}$$

wherein Step A$_4$) further comprises the following Step k):

k) a step of measuring respective albumin-derived absorbances (U) of the two or more sample materials to determine an albumin concentration (V);

Step $A_4$) further includes, after Steps j) and k), the following Step l):

l) a step of determining, from the GA concentrations$_{[L]}$ (T), respective GA values$_1$ (W) of the two or more sample materials according to the following Equation 8:

$$GA\ value_1\ (W) = (GA\ concentration_{[L]}\ (T))/(albumin\ concentration\ (V))$$

$$(Equation\ 8);$$

wherein the method further comprises the following steps:

F1') a step of determining, from the GA-derived absorbance (S) of the sample material, a GA concentration$_{[P]}$ (N') in the enzymatic method corresponding thereto by using a calibration curve prepared by using the reference material;

F2') a step of determining, from the albumin-derived absorbance (U) of the sample material used in Step F1'), an albumin concentration (O') corresponding thereto by using a calibration curve prepared by using the reference material;

F5') a step of determining, from the GA concentration$_{[P]}$ (N'), a GA concentration$_{[L]}$ (R') of the sample material according to the Regression equation VI; and

F6') a step of determining a GA value ($Q_3$') traceable to a certified GA value$_1$ (W) set for the sample material used in Step F1') according to the following Equation 7':

$$GA\ value\ (Q_3')\ of\ sample\ material = (GA\ concentration_{[L]}\ (R'))/(albumin$$

$$concentration\ (O'))\quad (Equation\ 7').$$

16. The method according to any one of Claims 1 to 15, wherein three or more GA certified reference materials are used.

17. The method according to any one of Claims 1 to 16, wherein the certified GA value is a certified GA value determined by isotope dilution mass spectrometry (ID-MS).

18. The method according to any one of Claims 1 to 16, wherein the certified GA value is a certified GA value determined by isotope dilution mass spectrometry (ID-MS) and the GA certified reference material is JCCRM 611.

19. A GA value measurement kit, comprising:

a regression equation selected from the group consisting of the Regression equation III determined by the method as claimed in Claim 2, the Regression equation IV determined by the method as claimed in Claim 7, the Regression equation V determined by the method as claimed in Claim 10, and the Regression equation VI determined by the method as claimed in Claim 13;

a GA measurement reagent;

an albumin measurement reagent; and

a reference material.

# Fig. 1

# Fig. 2

Fig. 3

# Fig. 4

Fig. 5

Fig. 6

Fig. 7

Fig. 8

## Fig. 9

Graph showing a linear relationship. X-axis: GA concentration$_{[P]}$(E) (µmol/L), ranging from 0 to 400. Y-axis: GA concentration$_{[L]}$(A) (µmol/L), ranging from 0.0 to 350.0. Equation: $y = 0.9998x + 33.783$

Fig. 10

$$y = 0.9961x + 47.467$$

# Fig. 11

Fig. 12

Fig. 13

## Fig. 14

## Fig. 15

Fig. 16

Fig. 17

Fig. 18

Fig. 19

Fig. 20

## INTERNATIONAL SEARCH REPORT

| International application No. |
| --- |
| PCT/JP2017/026014 |

### A. CLASSIFICATION OF SUBJECT MATTER

*G01N33/68*(2006.01)i, *C12M1/34*(2006.01)i, *G01N27/62*(2006.01)i, *G01N21/77*(2006.01)n

According to International Patent Classification (IPC) or to both national classification and IPC

### B. FIELDS SEARCHED

Minimum documentation searched (classification system followed by classification symbols)
G01N33/68, C12M1/34, G01N27/62, G01N21/77

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
| --- | --- | --- |
| A | Izumi TAKEI et al., "JSCC Recommended Method for Glycated Albumin Measurement in Serum" (Ver.1:2008-3-31), Japanese Journal of Clinical Chemistry, 30 April 2008 (30.04.2008), vol.37, no.2, pages 178 to 191 | 1–19 |
| A | JP 2006-149230 A (Asahi Kasei Pharma Corp.), 15 June 2006 (15.06.2006), entire text; all drawings (Family: none) | 1–19 |
| A | JP 2004-077457 A (Asahi Kasei Pharma Corp.), 11 March 2004 (11.03.2004), entire text; all drawings & JP 2006-64706 A & JP 2007-163515 A | 1–19 |

☒ Further documents are listed in the continuation of Box C. ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 05 October 2017 (05.10.17) | 17 October 2017 (17.10.17) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2017/026014

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2002/061119 A1  (Asahi Kasei Pharma Corp.), 08 August 2002 (08.08.2002), entire text; all drawings<br>& JP 2008-278898 A       & JP 2009-128 A<br>& JP 2009-129 A         & JP 2009-34110 A<br>& JP 2009-159989 A       & JP 4341809 B2<br>& JP 2011-45389 A        & JP 2011-155982 A<br>& US 2005/0101771 A1     & US 2011/0312009 A1<br>& US 2012/0129202 A1     & EP 1362925 A1<br>& EP 2107123 A2 | 1-19 |
| A | Izumi TAKEI et al., "Glycated Albumin (GA) Sokutei Hyojunka no Genkyo", The Japanese Journal of Clinical Pathology, 31 October 2007 (31.10.2007), vol.55 supplementary issue, page 285 | 1-19 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2002061119 A
- JP 2004077457 A

- JP 2001054398 A
- JP 2016148973 A

**Non-patent literature cited in the description**

- Establishment of reference procedure for GA measurement. Japan Society of Clinical Chemistry (JSCC)

- **TAKEI et al.** *Clinical Chemistry,* 2008, vol. 37, 178-191
- **GEISOW MJ.** Techniques in proteinchemistry. Academic Press, 1991, 576-572